# EUROPEAN PATENT APPLICATION

(11) **EP 4 458 431 A1**
(43) Date of publication of application: **06.11.2024**
(21) Application number: 22960339.4
(22) Date of filing: 30.09.2022
(51) Int. Cl.: A63B 22/02

(54) **METHOD FOR MONITORING RUNNING OF USER, AND SIGNAL ACQUISITION APPARATUS**

(71) Applicant: Shenzhen Shokz Co., Ltd., Shenzhen, Guangdong 518108 (CN)
(72) Inventor: ZHOU, Xin, Shenzhen, Guangdong 518108 (CN); SU, Lei, Shenzhen, Guangdong 518108 (CN); LI, Meiqi, Shenzhen, Guangdong 518108 (CN); ZHANG, Yuxiang, Shenzhen, Guangdong 518108 (CN); LIU, Jia, Shenzhen, Guangdong 518108 (CN); NING, Yixuan, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/123372
(87) International publication number: WO 2024/065720

(57) **Abstract**

Embodiments of the present disclosure provide a method for monitoring user's running and a signal collection device. The method may include: obtaining an electromyographic (EMG) signal of a leg muscle of a user when the user is running; identifying a plurality of feature values of the EMG signal in a plurality of target time intervals; and in response to that the plurality of feature values satisfy a preset condition, generating running feedback information.

## Description

### TECHNICAL FIELD

The present disclosure relates to the field of artificial intelligence technology, and in particular, to a method for monitoring user's running and a signal collection device.

### BACKGROUND

During the monitoring process of running, the collection of an electromyographic (EMG) signal of a leg muscle may be interfered with by a plurality of factors. For example, an artifact generated during movement, a difference in locations and structures of different leg muscles, etc., may increase the noise during the collection of the EMG signal. Additionally, how to monitor user's running based on the collected EMG signal is a problem to be solved.

Therefore, it is desirable to provide a method for monitoring user's running and a signal collection device. The signal collection device may be configured to collect an EMG signal of a leg muscle. The user's running can be monitored based on the collected EMG signal, which can improve the accuracy of signal collection, provide the user with running feedback information, and enhance user experience.

### SUMMARY

One or more embodiments of the present disclosure provide a signal collection device. The signal collection device includes a wearable body and a plurality of electrodes disposed on the wearable body and in contact with the skin of a user. The plurality of electrodes include a first electrode and a second electrode. The first electrode and the second electrode are configured to collect an electromyographic (EMG) signal of a muscle of the user and are spaced apart along a muscle fiber direction of the muscle.
The muscle refers to a single muscle or a muscle group (e.g., the quadriceps muscle group, which includes the rectus femoris, lateral femoris, medial femoris, and intermediate femoris). The first electrode and the second electrode extend along an extension direction perpendicular to the muscle fiber direction, respectively, and each of the first electrode and the second electrode has a size in the muscle fiber direction that is smaller than a size in the extension direction.

In some embodiments, the plurality of electrodes further include an artifact electrode spaced apart from the first electrode along the extension direction, and the signal collection device further includes a processing circuit configured to perform noise cancellation on a signal collected by the first electrode and a signal collected by the second electrode based on a signal collected by the artifact electrode.

In some embodiments, the performing the noise cancellation on the signal collected by the first electrode and the signal collected by the second electrode based on the signal collected by the artifact electrode includes: obtaining a first signal by performing a differential operation on the signal collected by the first electrode and the signal collected by the second electrode; obtaining a second signal by performing a differential operation on the signal collected by the first electrode and the signal collected by the artifact electrode; and performing the noise cancellation on the first signal based on the second signal.

In some embodiments, the plurality of electrodes further include a second artifact electrode spaced apart from the second electrode along the extension direction, and the processing circuit is further configured to perform the noise cancellation on the signal collected by the first electrode and the signal collected by the second electrode based on the signal collected by the artifact electrode and a signal collected by the second artifact electrode.

In some embodiments, the performing the noise cancellation on the signal collected by the first electrode and the signal collected by the second electrode based on the signal collected by the artifact electrode and the signal collected by the second artifact electrode includes: obtaining a first signal by performing a differential operation on the signal collected by the first electrode and the signal collected by the second electrode; obtaining a second signal by performing a differential operation on the signal collected by the first electrode and the signal collected by the artifact electrode; obtaining a third signal by performing a differential operation on the signal collected by the second electrode and the signal collected by the second artifact electrode; and performing the noise cancellation on the first signal based on the second signal and the third signal.

In some embodiments, a value of a configuration parameter of the artifact electrode is different from a value of the configuration parameter of the first electrode or a value of the configuration parameter of the second electrode.

In some embodiments, the configuration parameter includes at least one of an area, a material, or a pressure on the skin of the user per unit of area.

In some embodiments, the plurality of electrodes further include a reference electrode. The reference electrode is an integral electrode, and in the extension direction, a length of the reference electrode is greater than a length of the first electrode or a length of the second electrode.

In some embodiments, the plurality of electrodes further include a reference electrode including a plurality of sub-electrodes spaced apart and connected by wires, wherein, along the muscle fiber direction of the muscle, projections of the plurality of sub-electrodes are at least partially coincident with the first electrode or the second electrode.

In some embodiments, the wearable body includes a leg strap or a leg sleeve, and the plurality of electrodes are configured to collect an EMG signal of a front side or a back side of a leg of the user.

In some embodiments, the signal collection device further includes a plurality of additional electrodes configured to collect an EMG signal of an outer side of a thigh of the user. The plurality of additional electrodes are disposed above the plurality of electrodes along a height direction of the user.

In some embodiments, the EMG signal of the outer side of the thigh includes an EMG signal of a tensor fascia lata.

In some embodiments, a ratio of a height difference between the plurality of additional electrodes and the plurality of electrodes to a length of the thigh is greater than 1/10.

In some embodiments, the signal collection device further includes a circuit board disposed on the wearable body. The circuit board is provided with a processing circuit configured to process signals collected by the plurality of electrodes, wherein each of the plurality of electrodes is connected to the circuit board via a wire, the wearable body includes a two-layer structure, the wire is sandwiched in the two-layer structure, and a length of the wire is configured to adapt to an elastic stretching length of the wearable body.

In some embodiments, for each electrode of the plurality of electrodes, the wire includes a first connection point connected to the electrode and a second connection point connected to the circuit board, and the length of the wire is greater than 110% of a straight-line distance between the first connection point and the second connection point.

In some embodiments, the signal collection device is configured to collect an EMG signal of a leg muscle of the user, and the processing circuit is configured to: obtain the EMG signal of the leg muscle of the user when the user is running; identify a plurality of feature values of the EMG signal in a plurality of target time intervals; and in response to that the plurality of feature values satisfy a preset condition, generate running feedback information.

In some embodiments, the plurality of feature values include at least one of: amplitude values, absolute amplitude values, an average amplitude value, a maximum amplitude value, a minimum amplitude value, a medium value frequency, a peak frequency, a total force value, an EMG power, a foot contact time, or an average frequency, corresponding to the EMG signal in each of the plurality of target time intervals.

In some embodiments, the identifying a plurality of feature values of the EMG signal in a plurality of target time intervals includes: segmenting the EMG signal based on a plurality of first time windows and determining first segmented EMG signals in the plurality of first time windows; and determining the plurality of feature values based on the first segmented EMG signals.

In some embodiments, the determining the plurality of feature values based on the first segmented EMG signals includes: determining a candidate value of each first segmented EMG signal; and determining, from a plurality of candidate values corresponding to the first segmented EMG signals, candidate values that satisfy a condition as the plurality of feature values.

In some embodiments, a duration of each first time window of the plurality of first time windows is smaller than or equal to a running cycle of the user, and the candidate value of each first segmented EMG signal includes an average amplitude value of the each first segmented EMG signal.

In some embodiments, the determining, from a plurality of candidate values corresponding to the first segmented EMG signals, candidate values that satisfy a condition as the plurality of feature values include: segmenting the EMG signal based on a plurality of second time windows and determining second segmented EMG signals in the plurality of second time windows; and determining the plurality of feature values based on candidate values of the second segmented EMG signal in each second time window.

In some embodiments, a duration of each second time window is larger than a duration of each first time window.

In some embodiments, the determining the plurality of feature values based on candidate values of the second segmented EMG signal in each second time window includes: for each second time window, determining at least one of a maximum value, a minimum value, an intermediate value, an average value, and a weighted average value of the candidate values of the second segmented EMG signal in the second time window as the plurality of feature values.

In some embodiments, the determining the plurality of feature values based on the first segmented EMG signal includes: determining an average amplitude value of each first segmented EMG signal as the plurality of feature values.

In some embodiments, the preset condition includes: a feature parameter that indicates a change in the plurality of feature values being greater than a preset threshold.

In some embodiments, the plurality of feature values include a maximal value, and the preset condition includes: the plurality of feature values including a feature value greater than the maximal value.

In some embodiments, the running feedback information includes at least one of: a fatigue reminder, a running workout amount recommendation, or a target muscle strength training recommendation.

In some embodiments, the EMG signal includes a plurality of EMG signals corresponding to a plurality of leg muscles of the user, the plurality of feature values include: an amplitude ratio of EMG signals of at least two leg muscles of the plurality of leg muscles when performing a same running action, and the preset condition includes: a difference between at least two feature values in the plurality of feature values being greater than a difference threshold.

One or more embodiments of the present disclosure provide a method for monitoring user's running. The method includes: obtaining an electromyographic (EMG) signal of a leg muscle of a user when the user is running; identifying a plurality of feature values of the EMG signal in a plurality of target time intervals; and in response to that the plurality of feature values satisfy a preset condition, generating running feedback information.

In some embodiments, the plurality of feature values include at least one of: amplitude values, an absolute amplitude value, an average amplitude value, a maximum amplitude value, a minimum amplitude value, a medium value frequency, a peak frequency, a total force value, an EMG power, a foot contact time, or an average frequency corresponding to the EMG signal in each of the plurality of target time intervals.

In some embodiments, the identifying a plurality of feature values of the EMG signal in a plurality of target time intervals includes: segmenting the EMG signal based on a plurality of first time windows and determining a first segmented EMG signals in the plurality of first time windows; and determining the plurality of feature values based on the first segmented EMG signals.

In some embodiments, the determining the plurality of feature values based on the first segmented EMG signals includes: determining a candidate value of each segmented EMG signal in the first segmented EMG signals; and determining, from a plurality of candidate values corresponding to the first segmented EMG signals, candidate values that satisfy a condition as the plurality of feature values.

In some embodiments, a duration of each first time window of the plurality of first time windows is smaller than or equal to a running cycle of the user, and the candidate values of each segmented EMG signals include an average amplitude value of the each segmented EMG signal.

In some embodiments, the determining, from a plurality of candidate values corresponding to the first segmented EMG signals, candidate values that satisfy a condition as the plurality of feature values includes: segmenting the EMG signal based on a plurality of second time window and determining a second segmented EMG signals in the plurality of second time windows; and filtering candidate values of a segmented EMG signal in each second time window of the plurality of second time windows and determining candidate values that satisfy the condition as the plurality of feature values.

In some embodiments, a duration of the each second time window is larger than a duration of the each first time window.

In some embodiments, the determining the plurality of feature values based on candidate values of the second segmented EMG signal in each second time window includes: for each second time window, determining at least one of a maximum value, a minimum value, an intermediate value, an average value, and a weighted average value of the candidate values of the second segmented EMG signal in the second time window as the plurality of feature values.

In some embodiments, the determining the plurality of feature values based on the first segmented EMG signal includes: determining an average amplitude value of each first segmented EMG signal as the plurality of feature values.

In some embodiments, the preset condition includes: a feature parameter that indicates a change in the plurality of feature values being greater than a preset threshold.

In some embodiments, the plurality of feature values include a maximal value, and the preset condition includes: the plurality of feature values including a feature value greater than the maximal value.

In some embodiments, the running feedback information includes at least one of: a fatigue reminder, a running workout amount recommendation, or a target muscle strength training recommendation.

In some embodiments, the EMG signal includes a plurality of EMG signals corresponding to a plurality of leg muscles of the user, the plurality of feature values include: an amplitude ratio of EMG signals of at least two leg muscles of the plurality of leg muscles when performing a same running action, and the preset condition includes: a difference between at least two feature values in the plurality of feature values being greater than a difference threshold.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating a system for monitoring user's running according to some embodiments of the present disclosure;
FIG. 2 is a block diagram illustrating an exemplary signal collection device according to some embodiments of the present disclosure;
FIG. 3 is a schematic diagram illustrating an exemplary structure of an electrode module according to some embodiments of the present disclosure;
FIG. 4A is a schematic diagram illustrating another exemplary structure of an electrode module according to some embodiments of the present disclosure;
FIG. 4B is a schematic diagram illustrating an exemplary process of noise cancellation on a signal collected by a first electrode and a signal collected by a second electrode based on a signal collected by an artifact electrode according to some embodiments of the present disclosure;
FIG. 5A is a schematic diagram illustrating another exemplary structure of an electrode module according to some embodiments of the present disclosure;
FIG. 5B is a schematic diagram illustrating an exemplary process of noise cancellation on a signal collected by a first electrode and a signal collected by a second electrode based on a signal collected by an artifact electrode and a signal collected by a second artifact electrode according to some embodiments of the present disclosure;
FIG. 6 is a schematic diagram illustrating an exemplary structure of a signal collection device according to some embodiments of the present disclosure;
FIG. 7 is a schematic diagram illustrating another exemplary structure of a signal collection device according to some embodiments of the present disclosure;
FIG. 8 is a schematic diagram illustrating electromyographic (EMG) signals at a plurality of positions on a leg of a user according to some embodiments of the present disclosure;
FIG. 9 is a block diagram illustrating an exemplary device for monitoring user's running according to some embodiments of the present disclosure;
FIG. 10 is a flowchart illustrating an exemplary process for monitoring user's running according to some embodiments of the present disclosure;
FIG. 11 is a flowchart illustrating an exemplary process for identifying a plurality of feature values of an EMG signal and generating running feedback information according to some embodiments of the present disclosure;
FIG. 12 is a schematic diagram illustrating a curve showing a change in a plurality of feature values of an EMG signal of a leg muscle of a user over time;
FIG. 13 is a flowchart illustrating another exemplary process for identifying a plurality of feature values of an EMG signal and generating running feedback information according to some embodiments of the present disclosure;
FIG. 14 is a schematic diagram illustrating a curve showing a change in a plurality of feature values of an EMG signal of a leg muscle of a user over time; and
FIG. 15 is a schematic diagram illustrating curves showing changes in an EMG signal of the biceps femoris muscle and an EMG signal of the rectus femoris muscle of a user over time when the user is running.

### DETAILED DESCRIPTION

In order to more clearly illustrate the technical solutions of the embodiments of the present disclosure, the accompanying drawings to be used in the description of the embodiments will be briefly described below. Obviously, the accompanying drawings in the following description are only some examples or embodiments of the present disclosure, and that the present disclosure may be applied to other similar scenarios in accordance with these drawings without creative labor for those skilled in the art. Unless obviously acquired from the context or the context illustrates otherwise, the same numeral in the drawings refers to the same structure or operation.

It should be understood that "system," "device," "unit," and/or "module" as used herein is a way to distinguish between different components, elements, parts, sections, or assemblies at different levels. However, these words may be replaced by other expressions if they accomplish the same purpose.

As indicated in the present disclosure and in the claims, the singular forms "a," "an," and "the" may be intended to include the plural forms as well, unless the context clearly indicates otherwise. In general, the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.

Flowcharts are used in the present disclosure to illustrate the operations performed by the system according to some embodiments of the present disclosure. It should be understood that the operations described herein are not necessarily executed in a specific order. Instead, the operations may be executed in reverse order or simultaneously. Additionally, one or more other operations may be added to these processes, or one or more operations may be removed from these processes.

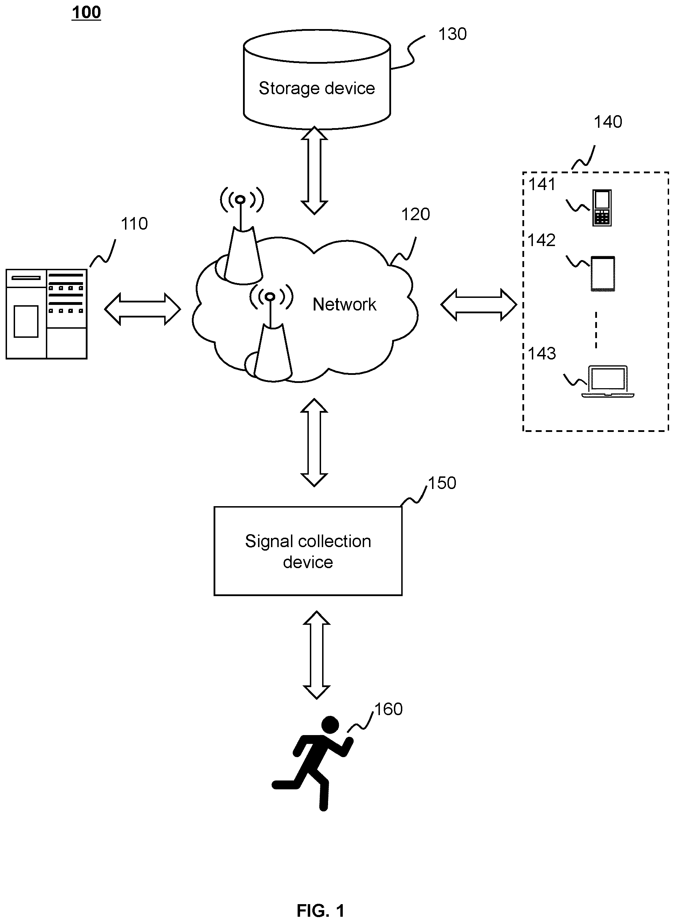

FIG. 1 is a schematic diagram illustrating a system for monitoring user's running according to some embodiments of the present disclosure.

According to FIG. 1, in some embodiments, a system 100 for monitoring user's running (hereinafter referred to as the system 100) may include a processing device 110, a network 120, a storage device 130, one or more terminal devices 140, and a signal collection device 150. Components of the system 100 may be connected in a variety of manners. For example, the signal collection device 150 may be connected to the storage device 130 and/or the processing device 110 via the network 120, or directly connected to the storage device 130 and/or the processing device 110. As another example, the storage device 130 may be directly connected to the processing device 110 or connected via the network 120. As another example, the one or more terminal devices 140 may be connected to the storage device 130 and/or the processing device 110 via the network 120 or may be directly connected to the storage device 130 and/or the processing device 110.

In some embodiments, the system 100 may generate running feedback information specific to a user's running status by implementing the methods and/or processes disclosed in the present disclosure. For example, the processing device 110 may collect a motion signal (e.g., an EMG signal) of the leg muscles of the user when the user is running, identify a plurality of feature values of the motion signal in a plurality of target time intervals, and generate running feedback information in response to that the plurality of feature values satisfy a preset condition.

The processing device 110 may process data and/or information obtained from the signal collection device 150, the storage device 130, the one or more terminal devices 140, and/or other components of the system 100. In some embodiments, the processing device 110 may obtain the motion signal of the user 160 from any one or more of the signal collection device 150, the storage device 130, or the one or more terminal devices 140, and process the motion signal to generate the running feedback information. In some embodiments, the processing device 110 may obtain pre-stored computer instructions from the storage device 130 and execute the computer instructions to implement a method for monitoring the user's running as described herein.

In some embodiments, the processing device 110 may be a single server or a server group. The server group may be centralized or distributed. In some embodiments, the processing device 110 may be local or remote. For example, the processing device 110 may access information and/or data from the signal collection device 150, the storage device 130, and/or the one or more terminal devices 140 via the network 120. As another example, the processing device 110 may be directly connected to the signal collection device 150, the storage device 130, and/or the one or more terminal devices 140 to access the information and/or data. In some embodiments, the processing device 110 may be implemented on a cloud platform. For example, the cloud platform may include a private cloud, a public cloud, a hybrid cloud, a community cloud, a distributed cloud, an inter-cloud cloud, a multi-cloud, or the like, or any combination thereof.

The network 120 may connect various components of the system 100 and/or connect the system 100 to an external resource component. The network 120 may enable communication between the various components of the system 100, as well as with other components of the system 100 outside of the system 100, thereby facilitating the exchange of data and/or information. For example, the processing device 110 may obtain a motion signal from the signal collection device 150 and/or the storage device 130 via the network 120. As another example, the processing device 110 may obtain a user operation instruction from the one or more terminal devices 140 via the network 120, and exemplary operation instructions may include, but are not limited to, setting user information (e.g., gender, age, height, weight, disease history, etc.), selecting a motion mode (e.g., running, jumping rope, swimming, muscle training, etc.), setting a motion time, etc.

In some embodiments, the network 120 may be any form of wired or wireless network, or any combination thereof. Merely by way of example, the network 120 may include a cable network, a wired network, a fiber optic network, a telecommunication network, an intranet, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc. or any combination thereof. In some embodiments, the network 120 may include at least one network access point, and at least one component of the system 100 may be connected to the network 120 via the access point to exchange data and/or information. For example, data such as motion signals, a user's motion status, or the like, may be communicated via the network 120.

The storage device 130 may store data, instructions, and/or any other information. In some embodiments, the storage device 130 may store data obtained from the signal collection device 150, the processing device 110, and/or the one or more terminal devices 140. For example, the storage device 130 may store motion signals acquired by the signal collection device 150. In some embodiments, the storage device 130 may store data and/or instructions used by the processing device 110 to execute or use to accomplish the exemplary methods described in the present disclosure. In some embodiments, the storage device 130 may include mass memory, removable memory, volatile read-write memory, read-only memory (ROM), or the like, or any combination thereof. Exemplary mass memory may include a disk, an optical disk, a solid state disk, or the like. In some embodiments, the storage device 130 may be implemented on a cloud platform. Merely by way of example, the cloud platform may include a private cloud, a public cloud, a hybrid cloud, a community cloud, a distributed cloud, an on-premises cloud, a multi-tiered cloud, or the like, or any combination thereof.

In some embodiments, the storage device 130 may be connected to the network 120 to communicate with at least one other component (e.g., the signal collection device 150, the processing device 110, the one or more terminal devices 140) of the system 100. At least one component of the system 100 may access data, instructions, or other information stored in the storage device 130 via the network 120. In some embodiments, the storage device 130 may be directly connected or in communication with one or more components (e.g., the signal collection device 150, the one or more terminal devices 140) of the system 100. In some embodiments, the storage device 130 may be part of the signal collection device 150 and/or the processing device 110.

The one or more terminal devices 140 may receive, send and/or display data. Received data may include data collected by the signal collection device 150, data stored by the storage device 130, running feedback information generated by the processing device 110, etc. For example, the data received and/or displayed by the one or more terminal devices 140 may include a motion signal collected by the signal collection device 150, a plurality of feature values of the motion signal in a plurality of target time intervals identified by the processing device 110, running feedback information generated by the processing device 110 based on the plurality of feature values of the motion signal, or the like. Sent data may include user input data, instructions, etc. For example, the one or more terminal devices 140 may send the operation instruction input by the user to the signal collection device 150 via the network 120 to control the signal collection device 150 to perform data collection.

In some embodiments, the one or more terminal devices 140 may include a mobile device 141, a tablet computer 142, a laptop computer 143, or the like, or any combination thereof. For example, the mobile device 141 may include a cell phone, a personal digital assistant (PDA), a medical mobile terminal, or the like, or any combination thereof. In some embodiments, the one or more terminal devices 140 may include an input device (e.g., a keyboard, a touch screen), an output device (e.g., a display, a speaker), or the like. In some embodiments, the processing device 110 may be part of the one or more terminal devices 140.

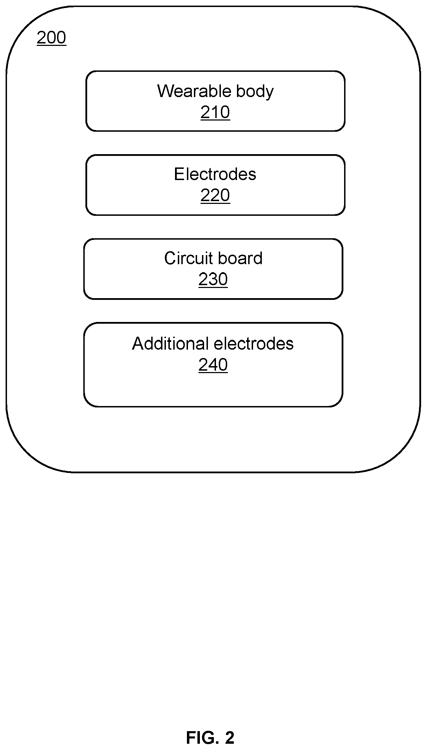

The signal collection device 150 refers to a device that collects a motion signal from a user. The motion signal refers to a signal generated by the user 160 during motion. Exemplary motion signals may include a postural signal, an electromyographic (EMG) signal, a mechanical signal, an electrocardiographic (ECG) signal, a respiratory signal, a sweat signal, or the like. In some embodiments, the signal collection device 150 may include a posture signal collection device, an EMG signal collection device, and a mechanics signal collection device. In some embodiments, the posture signal collection device may include a velocity sensor, an inertial sensor (e.g., an acceleration sensor, an angular velocity sensor (e.g., a gyroscope), etc.), an optical sensor (e.g., an optical distance sensor, a video/image grabber), an acoustic distance sensor, a tension sensor, or the like, or any combination thereof. In some embodiments, the EMG signal collection device may include one or more electrodes. For example, the EMG signal collection device may include a plurality of electrodes, and the plurality of electrodes may be configured to affix with different parts (e.g., the chest, back, elbow, leg, abdomen, wrist, etc.) of the user 160 to collect EMG signals from the different parts of the user 160. In some embodiments, the mechanical signal collection device may include a pressure sensor. For example, pressure sensors may be provided at different parts of the user 160 so as to collect pressure signals at the different parts. In some embodiments, the signal collection device 150 may also include an ECG signal collection device, a respiratory signal collection device, a sweat signal collection device, or the like. For example, the ECG signal collection device may include a plurality of electrodes, and the plurality of electrodes may be configured to affix with different parts of the user 160 to collect ECG signals of the user 160. As another example, the respiratory signal collection device may include a respiratory frequency sensor, a flow sensor, or the like, which are respectively configured to detect signals such as a respiratory frequency, a gas flow rate, or the like, of the user 160 in motion. As yet another example, the sweat signal collection device may include a plurality of electrodes in contact with the skin of the user 160, and the plurality of electrodes may be configured to detect a flow rate of the user's sweat, analyze a composition of the sweat, or the like. In some embodiments, the signal collection device 150 may have a separate power supply and may send collected data to other components (e.g., the processing device 110, the storage device 130, the one or more terminal devices 140) via wired or wireless (e.g., Bluetooth, WiFi, etc.) manners. In some embodiments, one or more components of the system 100 may be implemented on the signal collection device 150. For example, the processing device 110, the storage device 130, or the like may be included in the signal collection device 150. More descriptions regarding the structure of the signal collection device 150 may be found in FIG.2, FIG.3, FIG.4A, FIG.4 B, FIG.5 A, and FIG.5 B, and the related descriptions thereof.

The user 160 refers to a user of the signal collection device 150. For example, the user may be a runner, an exercise equipment tester, or the like. In some embodiments, the user may issue a query request. For example, the user inquires about his or her motion status, running feedback information, or the like. In some embodiments, the user may receive feedback (e.g., a query result, running feedback information, or the like) from the signal collection device 150 or the one or more terminal devices 140. In some embodiments, the count of users may be one or more.

It should be noted that the foregoing description of the system 100 is intended to be exemplary and illustrative only and does not limit the scope of the present disclosure. For those skilled in the art, various modifications and variations may be made to the system 100 under the guidance of the present disclosure. However, these modifications and variations remain within the scope of the present disclosure.

FIG. 2 is a block diagram illustrating an exemplary signal collection device according to some embodiments of the present disclosure.

The signal collection device 200 may be configured to collect an EMG signal of a muscle of a user. For example, the signal collection device 200 may be configured to collect the EMG signal of the muscle of the user via a plurality of electrodes affixed to the skin of the user. In some embodiments, the signal collection device 200 may be implemented as a wearable device (e.g., a garment), wherein a plurality of electrodes may be in contact with the skin of the user when the wearable device is worn by the user, thereby collecting the EMG signal from a surface of the skin. In some embodiments, the signal collection device 200 may be in contact with a plurality of parts of the human body, such as the calf, thigh, hip, waist, back, chest, shoulder, neck, or the like, to collect EMG signals of the plurality of parts. Merely by way of example, the signal collection device 200 may be configured to collect an EMG signal of a leg muscle of the user. As shown in FIG. 2, the signal collection device 200 may include a wearable body 210, a plurality of electrodes 220, a circuit board 230, and a plurality of additional electrodes 240.

The wearable body 210 refers to a carrier for carrying components such as the plurality of electrodes 220, the circuit board 230, and the plurality of electrodes 220. In some embodiments, the wearable body 210 may be configured as a leg-wearable device, such as a leg strap (e.g., a full leg strap, a thigh strap, a calf strap, etc.), a leg sleeve (e.g., a full leg sleeve, a thigh sleeve, a calf sleeve, etc.), a sock (e.g., a calf sock, a thigh sock, a pantyhose, etc.), a pair of pants, or the like.

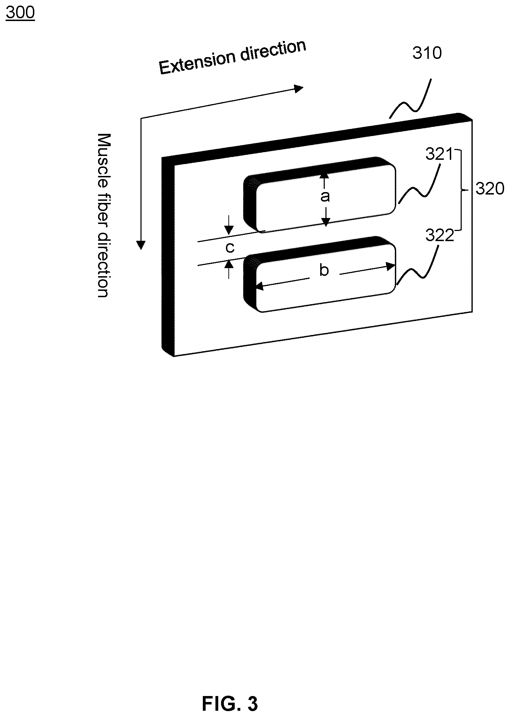

The plurality of electrodes 220 may be configured to collect an EMG signal of a muscle of the user. For example, the plurality of electrodes 220 may be secured to the wearable body 210 and in contact with the skin of the user to collect the EMG signal from the surface of the skin. In some embodiments, the plurality of electrodes 220 may include a first electrode and a second electrode. The first electrode and the second electrode may be configured to collect the EMG signal of a muscle of the user. The muscle may refer to a single muscle or a muscle group (e.g., a quadriceps muscle group including the rectus femoris, lateral femoris, medial femoris, and intermediate femoris). For example, the first electrode and the second electrode may be spaced apart along a muscle fiber direction of the muscle and extend along an extension direction perpendicular to the muscle fiber direction, respectively, so that the first electrode and the second electrode may be located at respective positions to collect potentials from the surface of the skin, and a potential difference between the collected potentials may reflect the EMG signal of the muscle. In some embodiments, the plurality of electrodes 220 may further include a reference electrode, the reference electrode may be configured to provide a reference potential for the first electrode or the second electrode, thereby reducing interference from noise. In some embodiments, the usability of the plurality of electrodes 220 may be improved by configuring parameters such as a material, a structure, a size, a spacing between electrodes, etc., of the first electrode and/or the second electrode. For example, the size of the first electrode and/or the second electrode, and the spacing between the first electrode and the second electrode may be configured to reduce noise or an effect on the strength of the EMG signal caused by a displacement of the plurality of electrodes 220 relative to the skin, thereby achieving more accurate and more efficient signal collection. As another example, the performance or comfort of the signal collection device 200 may be improved by configuring the material, the structure, etc., of the plurality of electrodes 220. More descriptions regarding configuring the parameters of the plurality of electrodes may be found in FIG. 3 and the related descriptions thereof, and will not be repeated here.

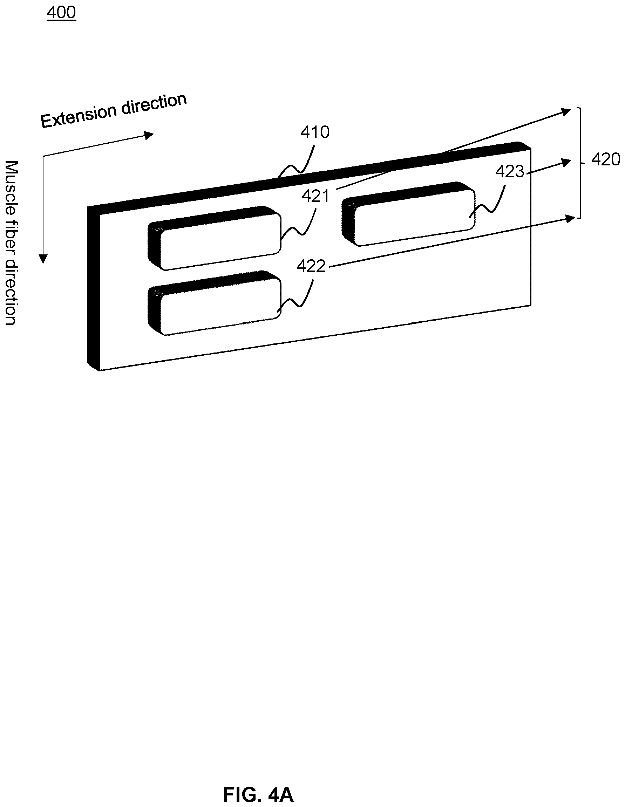

In some embodiments, the plurality of electrodes 220 may further include one or more artifact electrodes. The one or more artifact electrodes may be spaced apart with respect to the first electrode and/or the second electrode along the extension direction. In some embodiments, the one or more artifact electrodes may be used to eliminate noise from a signal collected by the first electrode and a signal collected by the second electrode. The noise may include a motion artifact noise caused by a displacement of the plurality of electrodes 220 relative to the skin, etc. By providing the one or more artifact electrodes, the processing device may process the signal collected by the first electrode and the signal collected by the second electrode based on a signal collected by the one or more artifact electrodes, thereby eliminating the effect of noise on the EMG signal, and obtaining an EMG signal with higher quality. More descriptions regarding the artifact electrode may be found in FIGs. 4 A-5B and the related descriptions thereof, which will not be repeated here.

The circuit board 230 may be secured to the wearing body 210, and the plurality of electrodes 220 may be respectively connected to the circuit board 230 by wires. The circuit board 230 may be provided with a processing circuit, and the processing circuit may be configured to process, store, transmit, etc., a signal. For example, the processing circuit may be configured to process a signal collected by a plurality of electrodes of the circuit board 230. In some embodiments, the processing device 110 in the system 100 may be implemented on the circuit board 230. For example, the processing circuit may perform as the processing device 110 to implement the method for monitoring the user's running as described in the present disclosure.

In some embodiments, the plurality of electrodes 220 may be configured to collect EMG signals from an anterior side (i.e., a front side), a posterior side (i.e., a back side), or a medial side (i.e., an inner side) of a leg part of the user. Taking the thigh as an example, muscles on the anterior side of the thigh of the user may include the quadriceps femoris, the sartorius, etc.; muscles on the posterior side of the thigh may include the biceps femoris, the semitendinosus, the semimembranosus, etc.; and muscles on the medial side of the thigh may include the gracilis, the adductor longus, the adductor magnus, the pectineus, etc. Since the iliotibial band on a lateral side (i.e., an outer side) of the thigh is a non-muscle tissue, it may produce little or no EMG signal. If one or more electrodes are provided on the lateral side of the thigh (e.g., at the same or a similar height as the height of the muscle on the anterior or posterior side of the thigh), an effective EMG signal may not be collected, and even if an EMG signal is collected, the collected EMG signal may be susceptible to interference from adjacent anterior or posterior muscles. However, postural errors or excessive fatigue during the user's motion (e.g., running, side leg raises, etc.) may cause iliotibial band syndrome. The iliotibial band therefore needs to be monitored during the monitoring of the user's motion. For example, since the iliotibial band is pulled by the tensor fascia lata of the hip, a condition of the iliotibial band may be determined based on an EMG signal of the tensor fascia lata. Therefore, in some embodiments, the signal collection device 200 may further include a plurality of additional electrodes 220 for collecting an EMG signal of the lateral side of the thigh of the user (e.g., an EMG signal of the tensor fascia lata). In some embodiments, since the tensor fascia lata is higher than the muscles on the anterior or posterior side of the thigh, to collect an effective EMG signal of the lateral side of the thigh while avoiding an influence of the muscles on the anterior or posterior side of the thigh, the plurality of additional electrodes 240 may be disposed above the plurality of electrodes 220 along a height direction of the user.

It should be noted that the above description of FIG. 2 is provided for illustrative purposes only and is not intended to limit the scope of the present disclosure. For those skilled in the art, a wide variety of variations and modifications may be made under the guidance of the present disclosure. For example, in some embodiments, the signal collection device 200 may include one or more components (e.g., batteries, Bluetooth, amplifiers, memory, inertial sensors, etc.). Optionally or additionally, the one or more components may be located on the lateral side of the leg or at the waist, etc., when the user is wearing the signal collection device 200. These variations and modifications do not depart from the scope of the present disclosure.

FIG. 3 is a schematic diagram illustrating an exemplary structure of an electrode module according to some embodiments of the present disclosure. In some embodiments, an electrode module 300 may include a substrate structure 310 and a plurality of electrodes 320, as shown in FIG. 3. The plurality of electrodes 320 may be secured to the wearable body by the substrate structure 310. For example, the substrate structure may be made of a flexible insulating material (e.g., resin, soft PVC, silicone, fabric, etc.), and the plurality of electrodes 320 may be fixedly attached to the substrate structure 310 by pasting, snap-fitting, welding, sewing, hot pressing, etc., and further fixed to the wearing body through the substrate structure 310. In some embodiments, the substrate structure 310 may be omitted, and the plurality of electrodes 320 may be integrally molded (e.g., integrally woven, etc.) with the wearable body. The plurality of electrodes 320 may be in contact with the skin of a user when the user is wearing the signal collection device. The plurality of electrodes 320 may include a first electrode 321 and a second electrode 322. The first electrode 321 and the second electrode 322 may be configured to collect an EMG signal of a muscle of the user. For example, the first electrode 321 and the second electrode 322 may be proximate to an intermediate position of the muscle in a muscle fiber direction (also referred to as a muscle length direction) when the user wears the signal collection device. The first electrode 321 and the second electrode 322 may be spaced apart along the muscle fiber direction and extend in an extension direction perpendicular to the muscle fiber direction, respectively. Different positions in the muscle fiber direction have different electric potentials, a position of a muscle fiber where the first electrode 321 is located has a first electric potential, a position of a muscle fiber where the second electrode 322 is located has a second electric potential, the first potential and the second potential have a potential difference between them, and the potential difference may be used to reflect the EMG signal.

In some embodiments, a collection accuracy and/or a collection efficiency of the plurality of electrodes 320 may be improved by configuring dimensions of the first electrode 321 and/or the second electrode 322, spacing between the first electrode 321 and the second electrode 322, or the like, to collect a signal more accurately and more efficiently. As shown in FIG. 2, a width of the first electrode 321 and/or the second electrode 322 in the muscle fiber direction is a, a length in the extension direction is b, and the spacing between the first electrode 321 and the second electrode 322 (i.e., a distance between two adjacent sides of the first electrode 321 and the second electrode 322) is c. In some embodiments, the length b of the first electrode 321 and/or the second electrode 322 may be determined based on the size of the muscle (also referred to as a muscle width) in the extension direction of the electrodes. For example, the length *b* may have a large value to minimize noise caused when there is a relative displacement between the plurality of electrodes 320 and the muscle, or an effect on a strength of the collected EMG signal when the plurality of electrodes 320 are deviated from a position of the muscle. Merely by way of example, the length *b* may be greater than or equal to the width of the muscle, greater than or equal to 30% of the width of the muscle, 50% of the width, 60% of the width, 80% of the width, etc. As another example, when the length b is too large, the plurality of electrodes 320 may span two or more groups of muscles (e.g., a group of target muscles and a group of non-target muscles), causing collected EMG signals to interfere with each other, and further making it impossible to accurately detect the EMG signal of the target muscle. Therefore, the length *b* may not be too large. Merely by way of example, the length *b* may be greater than or equal to 30% of the width of the target muscle and less than or equal to a sum of the width of the target muscle and a spacing between the target muscle and the non-target muscle. As another example, the length *b* may be greater than or equal to 50% of the width of the target muscle and less than or equal to the width of the target muscle. In some embodiments, for each target muscle of the group of target muscles, a length b of an electrode 320 corresponding to the target muscle may be determined based on test data of the target muscle, and a scaling factor of the target muscle may be determined based on a relative relationship (e.g., a size relationship) of the target muscle to other target muscles of the group of target muscles, thereby determining the length b of the electrode 320 corresponding to the target muscle. Taking the rectus femoris muscle as an example, a range of the length b of the corresponding electrode 320 may be determined based on the test data of the rectus femoris muscle. For example, the length b corresponding to the rectus femoris muscle may range from 1 mm to 120 mm.
As another example, the length b corresponding to the rectus femoris muscle may range from 10 mm to 100 mm. As yet another example, the length b corresponding to the rectus femoris muscle may range from 20 mm to 80 mm. Furthermore, different scaling factors may be determined for different target muscles. Merely by way of example, the size of the biceps femoris muscle is similar to the size of the rectus femoris muscle, and the scaling factor for the biceps femoris muscle may be in the range of 0.8 to 1 (e.g., 0.9). For example, the length b of the electrode 320 corresponding to the biceps femoris muscle may range from 0.9 mm to 108 mm. As another example, the length b of the electrode 320 corresponding to the biceps femoris muscle may range from 9 mm-90 mm. As another example, the length b of the electrode 320 corresponding to the biceps femoris muscle may range from 18 mm-72 mm. As another example, the scaling factor for the tensor fasciae lata may range from 0.5 to 0.8. As another example, the scaling factor for the tibialis anterior may range from 0.3 to 0.7. As yet another example, the scaling factor for the gastrocnemius may range from 0.4 to 0.8.

In some embodiments, the strength of the EMG signal collected by the plurality of electrodes 320 may be positively correlated with the spacing c between the first electrode 321 and the second electrode 322. Thus, the spacing c may not be too small to avoid affecting the strength of the EMG signal. When the spacing c is too large, inconsistency between the first electrode 321 and the second electrode 322 may be caused, which increases the noise in the EMG signal, and an excessively large spacing c may exceed a muscle range. Therefore, the spacing c between the first electrode 321 and the second electrode 322 may be set within a preset range. For example, the spacing c may be in a range of 1 mm-50 mm. As another example, the spacing c may be in a range of 5 mm-40 mm. As yet another example, the spacing c may be in a range of 10 mm-30 mm.

In some embodiments, to avoid a reduction in the strength of the EMG signals caused by the cancellation of EMG signals at end positions of the plurality of electrodes 320, the width a and/or the spacing c of each electrode of the plurality of electrodes 320 may be set in a preset range. For example, the end positions may be two ends (i.e., two ends of a single electrode that form the width a) of the single electrode along the muscle fiber direction. Correspondingly, the width a of the single electrode needs to be within the preset range. As another example, the end positions may be two ends of the plurality of electrodes 320 as a whole in the muscle fiber direction (i.e., an end of the first electrode 321 and an end of the second electrode 322 away from each other). Correspondingly, the width (i.e., 2a+c) of the plurality of electrodes 320 as a whole may be within a preset range. In some embodiments, the preset range may be related to a target frequency (e.g., 0-500 Hz, 10 Hz -300 Hz, 30 Hz -200 Hz, 60 Hz -140 Hz, etc.) at which the cancellation needs to be avoided. For example, the width a of a single electrode may be less than a half-wavelength of an EMG signal of a target frequency. As another example, the width 2a+c of the plurality of electrodes 320 as a whole may be less than the full wavelength of the EMG signal of the target frequency.

According to the above description relating to the dimensions of the plurality of electrodes 320, the length b of the first electrode 321 or the second electrode 322 is relatively large and the width a is relatively small. In some embodiments, the length b of the first electrode 321 or the second electrode 322 in the muscle fiber direction may be greater than the width a of the first electrode 321 or the second electrode 322 in the extension direction (i.e., b/a ≥ 1).

In some embodiments, the performance or comfort of the signal collection device 200 may be improved by configuring a material, a structure, or the like, of the plurality of electrodes 320. For example, the plurality of electrodes 320 may be waterproof, which may prevent short-circuiting between electrodes. As another example, the plurality of electrodes 320 may be made of a resilient material so that the signal collection device may be put on and taken off more easily, thereby improving the comfort of the signal collection device. Merely by way of example, an elasticity of the material of the plurality of electrodes 320 may be in a range of 0% to 200%. As another example, the plurality of electrodes 320 may be durable so as to withstand multiple washing machine loads, repeated wearing and taking off, skin friction, or the like without affecting other properties. As yet another example, the plurality of electrodes 320 may have a corrosion resistance (e.g., resistant to sweat corrosion). As yet another example, the plurality of electrodes 320 may have a good electrical conductivity. Merely by way of example, the plurality of electrodes 320 may have a conductivity greater than 10K Ω/cm, and a change in the conductivity of the plurality of electrodes 320 under deformation or other influences may be less than 10K Ω or less than 20% of an original conductivity. As another example, the plurality of electrodes 320 may be breathable. Merely by way of example, the plurality of electrodes 320 may be made of a breathable material (e.g., conductive fabric). As another example, the plurality of electrodes 320 may have a breathable structure (e.g., perforations, or gas channels in a middle layer). Furthermore, the plurality of electrodes 320 may have a certain degree of softness so as to ensure the comfort of the signal collection device. As another example, the plurality of electrodes 320 may be configured to protrude towards a surface of the skin with respect to the substrate structure 310 (or the wearing body), so that a localized pressurization effect may be achieved to promote the fit of the plurality of electrodes 320 to the surface of the skin. Merely by way of example, a protrusion height of the plurality of electrodes 320 may be in a range of 0-10 mm. As another example, a filler may be provided between the plurality of electrodes 320 and the substrate structure 310 to achieve the protrusion of the plurality of electrodes 320. A material of the filler (e.g., a sponge, etc.) may have properties such as preset resilience, preset softness, preset water retention capacity, or the like. The preset resilience and preset softness ensure that the signal collection device is comfortable when worn. The preset water retention capacity may reduce the accumulation of excessive water on the surface of the skin while storing part of the water to guarantee the electrodes and the skin to have good contact for a long time.

FIG. 4A is a schematic diagram illustrating another exemplary structure of an electrode module according to some embodiments of the present disclosure. In some embodiments, an electrode module 400 may include a substrate structure 410 and a plurality of electrodes 420, as shown in FIG. 4 A. The plurality of electrodes 420 may include a first electrode 421, a second electrode 422, and an artifact electrode 423. In some embodiments, the first electrode 421 and the second electrode 422 may be the same as or similar to the first electrode 321 and the second electrode 322, respectively, as illustrated in FIG. 3. For example, the first electrodes 421 and the second electrodes 422 may be spaced apart along a muscle fiber direction and configured to collect an EMG signal of a muscle of a user.

In some embodiments, the EMG signal collected by the electrode module 400 may contain a motion artifacts (MA) noise. The MA noise is a noise caused by motion. For example, the motion causes a change in the fit or position of the electrodes, increasing the noise in the collected EMG signal. As another example, the motion causes fluctuations in the potential of the stratum corneum, increasing the noise in the collected EMG signal. The artifact electrode 423 may be configured to collect a motion artifact signal, thereby removing the noise from the EMG signal. For example, a potential difference between potentials collected by the first electrode 421 and the second electrode 422 without an influence of motion may be expressed as a raw EMG signal E. The fluctuations in the potential of the stratum corneum caused by motion may result in a motion artifact signal M1 in the signals collected by the first electrode 421 and the second electrode 422. In other words, under the influence of motion, the EMG signal collected by the first electrode 421 and the second electrode 422 is a composite signal of E and M1. As shown in FIG. 4 A, the artifact electrode 423 may be spaced apart with respect to the first electrode 421 along an extension direction of the first electrode 421. In such cases, the artifact electrode 423 is located at the same potential of the muscle as the first electrode 421, and the potential difference between the artifact electrode 423 and the first electrode 421 is almost 0.
Thus, the signal collected by the artifact electrode 423 and the first electrode 421 may be a motion artifact signal M2 due to motion. In some embodiments, M2 and M1 may be motion artifact signals caused by a motion at the same moment, and thus have the same or similar characteristics (e.g., change trends, etc.). Therefore, the composite signal of E and M1 collected by the first electrode 421 and the second electrode 422 may be analyzed and processed based on M2 so as to obtain the raw EMG signal E.

In some embodiments, the artifact electrode 423 may be fixed relative to the first electrode 421 and/or the second electrode 422 during movement, thereby increasing a linkage between the artifact electrode 423 and the first electrode 421 and/or the second electrode 422, and avoiding an effect of a relative displacement between the artifact electrode 423 and the first electrode 421 and/or the second electrode 422 on a collection result. For example, the artifact electrode 423 may be connected to the first electrode 421 and/or the second electrode 422 using a connector with a relatively small elasticity. As another example, the artifact electrode 423 and the first electrode 421 and/or the second electrode 422 may be provided on a substrate structure with a relatively small elasticity.

In some embodiments, the amplitude of the raw EMG signal directly collected by the electrodes is usually relatively small, and a processing circuit of the signal collection device may process (e.g., perform a differential amplification operation, etc. on) the amplitude of the raw EMG signal to obtain an output EMG signal. In such cases, the value of a configuration parameter of the artifact electrode 423 may be different from the value of a configuration parameter of the first electrode 421 (or the second electrode 422) so that a difference between the signal collected by the artifact electrode 423 and the signal collected by the first electrode 421 may be increased after the differential amplification operation, thereby increasing strength of the motion artifact signal. For example, the material of the artifact electrode 423 and the material of the first electrode 421 may be different. As another example, an area of the artifact electrode 423 and an area of the first electrode 421 may be different. Merely by way of example, a ratio of a difference between the area of the artifact electrode 423 and the area of the first electrode 421 to the area of the first electrode 421 may be no less than 5%. As another example, the pressure of the artifact electrode 423 on the skin of the user per unit area and the pressure of the first electrode 421 on the skin of the user per unit area may be different. Merely by way of example, a ratio of a difference between the pressure per unit area of the artifact electrode 423 and the pressure per unit area of the first electrode 421 to the pressure per unit area of the first electrode 421 may be no less than 5%.

In some embodiments, the artifact electrode 423 may be arranged at any position on the substrate structure 410. For example, for uneven skin surfaces, spacing the artifact electrode 423 and the first electrode 421 along the extension direction may prevent the artifact electrode 423 from fully contacting the skin surface, thereby affecting signal acquisition. Therefore, the position of the artifact electrode 423 relative to the first electrode 421 may be adjusted. As another example, the artifact electrode 423 may not be in the extension direction of the first electrode 421, so as to achieve a differentiated configuration of the positions of of the artifact electrode 423 and the first electrode 421, which may increase the strength of the motion artifact signal. Merely by way of example, the artifact electrode 423 may be positioned above or below the first electrode 421 in the muscle fiber direction. In some embodiments, the position of the artifact electrode 423 may be further determined based on a linkage between the artifact electrode 423 and the first electrode 421 and/or the second electrode 422. For example, the differentiated configuration of the positions of the artifact electrode 423 and the first electrode 421 and/or the second electrode 422 may affect the linkage between the artifact electrode 423 and the first electrode 421 and/or the second electrode 422 (e.g., a difference between M1 and M2 under a same motion is significant). In such cases, the artifact electrode 423 may be provided at a suitable position so that M2 is as close as possible to M1 when the user performs a passive motion (i.e., the muscle does not generate EMG signals), and the position of the artifact electrode 423 at this time may be determined as a position that does not affect the linkage.

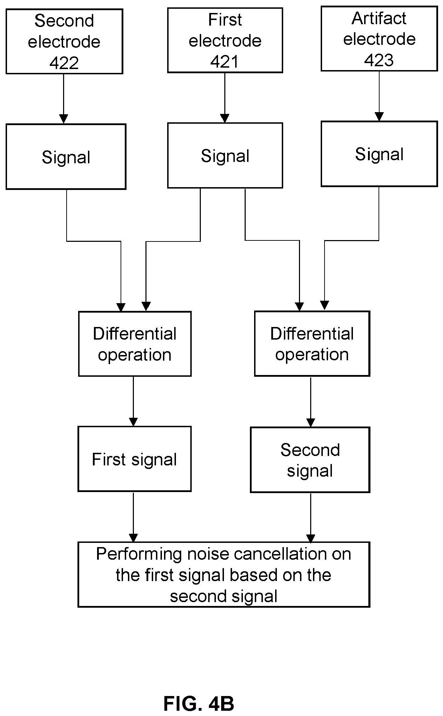

FIG. 4B is a schematic diagram illustrating an exemplary process of noise cancellation on a signal collected by a first electrode and a signal collected by a second electrode based on a signal collected by an artifact electrode according to some embodiments of the present disclosure.

In some embodiments, the noise cancellation process described in FIG. 4B may be performed by a processing circuit of a signal collection device. In some embodiments, the noise cancellation process described in FIG. 4B may also be performed by other devices (e.g., the processing device 110 of the system 100). As shown in FIG. 4B, the processing circuit may obtain a first signal (i.e., a composite signal of E and M1) by performing a differential operation on a signal collected by the first electrode 421 and a signal collected by the second electrode 422, and obtain a second signal (i.e., M2) by performing a differential operation on the signal collected by the first electrode 421 and a signal collected by the artifact electrode 423. Further, the processing circuit may perform noise cancellation on the first signal based on the second signal.

It is to be understood that the structure for noise cancellation and the noise cancellation process described in FIG. 4 A and FIG. 4 B are illustrative only. In some embodiments, the artifact electrode 423 may be the same or similar to the first electrode 421 and/or the second electrode 422. For example, the artifact electrode 423 may be a third electrode configured to cooperate with other electrodes (not shown in the drawing) for collecting an EMG signal of a muscle, or the third electrode may be configured to cooperate with the first electrode 421 or the second electrode 422 for noise cancellation on the signal collected by the first electrode 421 and the signal collected by the second electrode 422. In some embodiments, the noise cancellation may also be performed based on other structures and/or methods. For example, a contact impedance is formed when each electrode is in contact with the skin, and the pressure, position, etc. between the electrode and the skin changes when the human body is in motion, which causes fluctuations in the contact impedance, thereby affecting accuracy of the EMG signal. The fluctuations in the contact impedance may be used to characterize the motion artifact signal. Therefore, the signal collection device may include a component or a processing circuit for acquiring the contact impedance, so that the noise cancellation may be achieved based on the fluctuation of the contact impedance.

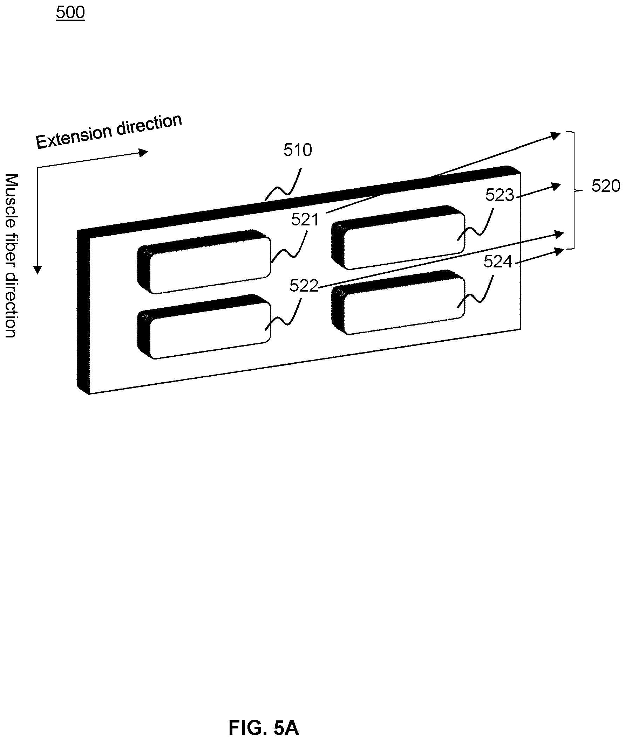

FIG. 5A is a schematic diagram illustrating another exemplary structure of an electrode module according to some embodiments of the present disclosure. In some embodiments, an electrode module 500 may include a substrate structure 510 and a plurality of electrodes 520, as shown in FIG. 5A. The plurality of electrodes 520 may include a first electrode 521, a second electrode 522, an artifact electrode 523, and a second artifact electrode 524. In some embodiments, the first electrode 521, the second electrode 522, and the artifact electrode 523 may be the same as or similar to the first electrode 421, the second electrode 422, and the artifact electrode 423, respectively, as described in FIGs. 4A and 4B. For example, the first electrode 521 and the second electrode 522 may be spaced apart along a muscle fiber direction and configured to collect an EMG signal of a muscle of a user. The artifact electrode 523 may be spaced relative to the first electrode 521 along an extension direction of the first electrode 521.

In some embodiments, the second artifact electrode 524 may be configured to collect an artifact signal, thereby removing noise from the EMG signal. For example, the first electrode 521 and the second electrode 522 may be configured to collect an EMG signal (e.g., a composite signal of the raw EMG signal E and the artifact signal M1), and the artifact electrode 523 and the second artifact electrode 524 may be configured to cooperate with the first electrode 521 and the second electrode 522 to collect an artifact signal, respectively. For example, the artifact electrode 523 may cooperate with the first electrode 521 to determine an artifact signal M2. The second artifact electrode 524 may cooperate with the second electrode 522 to determine an artifact signal M2'. A processing circuit may analyze and process the composite signals of E and M1 based on M2 and M2' to obtain the raw EMG signal E. In some embodiments, the artifact electrode 523 and the second artifact electrode 524 may be further configured to collect another EMG signal (e.g., a composite signal of a raw EMG signal E' and an artifact signal M1' at another position). The processing circuit may analyze and process the composite signal of E and M1 based on at least two of M2, M2', and the another EMG signal to obtain the raw EMG signal E, thereby further improving the efficiency and accuracy of the noise cancellation. Optionally or additionally, the first electrode 521 and the second electrode 522 may be configured to collect an EMG signal of a target muscle, and the artifact electrode 523 and the second artifact electrode 524 may be configured to collect an EMG signal of a non-target muscle. In such cases, the raw EMG signal E' at the another position has a relatively small value, and the another EMG signal may mainly include the artifact signal M1 ', so as to reduce an interference of the raw EMG signal E' in the noise cancellation process, thereby improving the efficiency and accuracy of the noise cancellation.

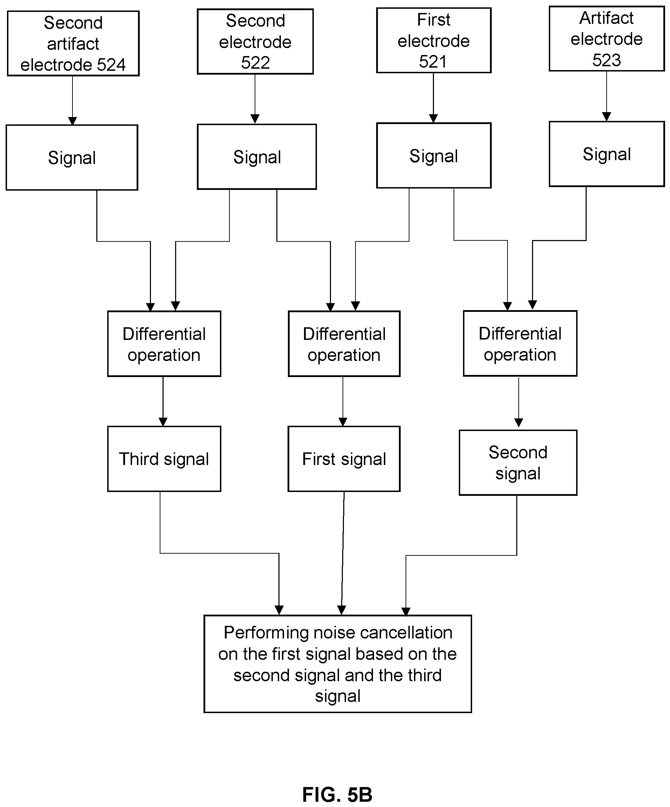

FIG. 5B is a schematic diagram illustrating an exemplary process of noise cancellation on a signal collected by a first electrode and a signal collected by a second electrode based on a signal collected by an artifact electrode and a signal collected by a second artifact electrode according to some embodiments of the present disclosure.

In some embodiments, the noise cancellation process described in FIG. 5B may be performed by a processing circuit corresponding to the electrode module 500. In some embodiments, the noise cancellation process described in FIG. 5B may also be performed by other devices (e.g., the processing device 110 of the system 100). According to FIGs. 5A and 5B, the processing circuit may obtain a first signal (i.e., a composite signal of E and M1) by performing a differential operation on a signal collected by the first electrode 521 and a signal collected by the second electrode 522, obtain a second signal (i.e., M2) by performing a differential operation on the signal collected by the first electrode 521 and a signal collected by the artifact electrode 523, and obtain a third signal (i.e., M2') by performing a differential operation on the signal collected by the second electrode 522 and a signal collected by the second artifact electrode 524. Further, the processing circuit may perform noise cancellation on the first signal based on the second signal and the third signal. Combining the third signal with the second signal for noise cancellation can introduce more reference information in the analysis and processing process, which can improve the efficiency and accuracy of the noise cancellation. In some embodiments, the processing circuit may also obtain a fourth signal (i.e., a composite signal of E' and M1') by performing a differential operation on the signal collected by the artifact electrode 523 and the signal collected by the second artifact electrode 524, and perform noise cancellation on the first signal based on at least two of the second signal, the third signal, and the fourth signal, thereby further improving the efficiency and accuracy of the noise cancellation.

In some embodiments, the artifact electrode 523 and the second artifact electrode 524 may be configured to collect EMG signals. For example, the first electrode 521 and the second electrode 522 may be configured as a first set of electrodes to collect a first EMG signal (i.e., a composite signal of E and M1), and the artifact electrode 523 and the second artifact electrode 524 may be configured as a second set of electrodes to collect a second EMG signal (i.e., a composite signal of E' and M1'). Additionally, the artifact electrode 523 may be configured to cooperate with the first electrode 521 to determine a first artifact signal M2, and the second artifact electrode 524 may be configured to cooperate with the second electrode 522 to determine a second artifact signal M2'. The processing circuit may perform the noise cancellation based on the first EMG signal, the second EMG signal, the first artifact signal, and the second artifact signal M2'. The plurality of signals may introduce additional reference information during analysis and processing, thereby further improving the efficiency and accuracy of the noise cancellation.

In some embodiments, the second artifact electrode 524 (or the artifact electrode 523) may be used as a reference electrode for providing a reference potential for the first electrode 521, the second electrode 522, and the artifact electrode 523.

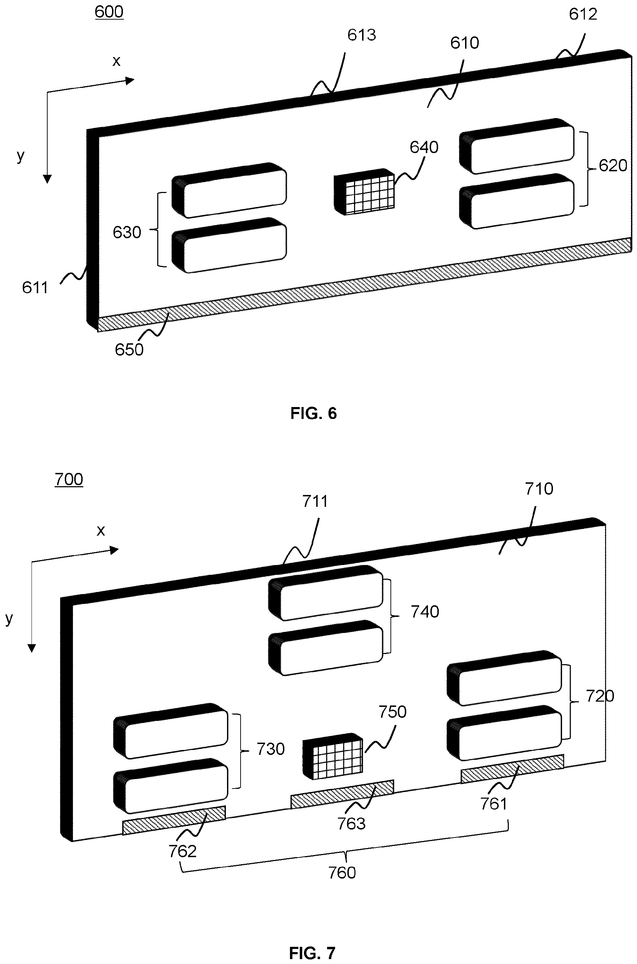

FIG. 6 is a schematic diagram illustrating an exemplary structure of a signal collection device according to some embodiments of the present disclosure.

As shown in FIG. 6, a signal collection device 600 may include a wearable body 610, a first electrode module 620, a second electrode module 630, a circuit board 640, and a reference electrode 650.

The wearable body 610 may be configured to carry the first electrode module 620, the second electrode module 630, the circuit board 640, and the reference electrode 650. In some embodiments, the first electrode module 620 and the second electrode module 630 may be the same as or similar to the electrode modules (e.g., the electrode module 300, the electrode module 400, the electrode module 500) described in FIGs. 3-5A, which will not be repeated here.

As shown in FIG. 6, the wearable body 610 may be configured as a leg strap (e.g., a full leg strap, a thigh strap, a calf strap, etc.). The first electrode module 620 and the second electrode module 630 may be secured to the wearable body 610 and configured to collect EMG signals from different muscles of a leg. Taking the wearable body 610 configured as a thigh strap as an example, the signal collection device 600 may be worn on the thigh of a user in a strapped manner. For example, two sides 611 and 612 of the wearable body 610 may include straps, interlocking Velcro, buttons, or the like, so that the wearable body 610 may be secured relative to the thigh of the user. When the user wears the signal collection device 600, the width direction (i.e., a y-direction) of the wearable body 610 may be substantially parallel to the height direction of the user. An upper lateral edge 613 may be located at the top (e.g., closer to the root of the thigh) of the wearable body 610, and the first electrode module 620 and the second electrode module 630 may be configured to collect EMG signals from the anterior (e.g., rectus femoris) and posterior (e.g., biceps femoris) muscles of the user's leg, respectively. In some embodiments, the wearable body 610 may further include an anti-slip strip (not shown in the drawing) configured to prevent the wearable body 610 from sliding relative to the skin of the thigh. For example, the anti-slip strip may be provided on the inner side of the wearable body 610, close to the upper lateral edge 613, which prevents sweat from flowing from the upper lateral edge 613 into the wearable body 610 and prevents slipping of the wearable body 610. In some embodiments, the wearable body 610 may further include positioning marks (not shown in the drawing) so that the user may adjust the electrode module to a correct muscle position according to the positioning marks. In some embodiments, the wearable body 610 may have a preset stretch coefficient in a length direction (i.e., x direction) of the wearable body 610 so that the wearable body 610 may be elastically stretched in the x direction to fit snugly against the skin of the thigh. The stretch coefficient refers to a ratio of the length of the wearable body 610 after elastic stretching to the length of the wearable body 610 in an unstretched state. Correspondingly, the length of the wearable body 610 in the unstretched state may be determined based on the preset stretch coefficient and a region where the wearable body 610 is to be worn. For example, if the region where the wearable body 610 is to be worn is the thigh, with an upper dimension of 56 cm, and the preset stretch coefficient is 20%, the length of the wearable body 610 may have an upper dimension of 46 cm.

The circuit board 640 may be secured to the wearable body 610. The circuit board 640 may be provided with a processing circuit, and the processing circuit may be configured to process signal and/or data, or the like. For example, the processing circuit may perform differentiation, gaining, filtering, conversion, storage, transmission operations, etc., on signals collected by the electrodes. As another example, the signal collection device 600 may also include one or more other components (e.g., batteries, Bluetooth, memory, inertial sensors, etc.). The processing circuit may also process (e.g., charging, voltage stabilization, etc.) signals and/or data associated with the one or more other components.

In some embodiments, the processing circuit may be configured to process signals collected by the first electrode module 620, the second electrode module 630, and the reference electrode 650, wherein each electrode of the first electrode module 620, the second electrode module 630, and the reference electrode 650 may be respectively connected to the circuit board 640 via wires (not shown in the drawing). In some embodiments, two wires connecting the same electrode may be located close to each other to reduce the area of a region between the two wires. For example, the two wires may be intertwined with each other and extend toward the circuit board 640. In some embodiments, the wire may be made of a conductive material, such as copper wire, silver wire, fabric fibers of silver nanoparticles, etc. In some embodiments, the wire and the electrodes connected thereto may be a one-piece structure made of the same material. For example, the wire and the electrode connected thereto may be obtained by integrally cutting from a conductive film. As another example, the wire and the electrode connected thereto may be a one-piece structure formed by spraying a conductive material. Portions of the one-piece structure other than a skin-fitting surface of the electrode may be waterproof and insulated. In some embodiments, the circuit board 640 may further include wires connected to one or more other components for processing or exchanging data, electrical energy, or the like. In some embodiments, the wire may be resilient to adapt to an elastic stretching length of the wearable body 610 when worn. For example, the wire may be made of an elastic material. As another example, a structure or a length of the wire may be configured to adapt to the elastic stretching length of the wearable body 610 when worn. Merely by way of example, the wire may include a first connection point connected to one electrode and a second connection point connected to the circuit board 640. The length of the wire may be greater than 110%, 120%, 140%, 180%, etc., of a straight-line distance between the first connection point and the second connection point, such that the wire may be adapted to a stretched length of the wearable body 610, thereby avoiding the influence of the elastic stretching of the wearable body 610 on the conductivity and structural stability when the length of the wire is too short.

In some embodiments, the signal collection device 600 may also include a circuit board 640 and a fixing module for one or more other components. For example, the fixing module may include an elastic cloth pouch, and the elastic cloth pouch may be secured to the wearable body 610 for accommodating the circuit board 640 and the one or more other components. As another example, the wearable body 610 may include a two-layer structure, and the circuit board 640 and the one or more other components may be provided between the two-layer structure.

The reference electrode 650 may be configured to provide a reference potential for the first electrode module 620 and the second electrode module 630. In some embodiments, the reference electrode 650 may be provided at a location where the EMG signal is not obvious, thereby preventing the reference potential from being too large and affecting the collection of the EMG signal. For example, the reference electrode 650 may be provided at an end of a muscle (e.g., a location on the thigh near the knee or the root of the thigh). In some embodiments, the reference electrode 650 may be an integral electrode disposed below the wearable body 610, as shown in FIG. 6, and the length of the integral electrode may be greater than lengths of electrodes of the first electrode module 620 and lengths of electrodes of the second electrode module 630.

FIG. 7 is a schematic diagram illustrating another exemplary structure of a signal collection device according to some embodiments of the present disclosure.

As shown in FIG. 7, a signal collection device 700 may include a wearable body 710, a first electrode module 720, a second electrode module 730, a third electrode module 740, a circuit board 750, and a reference electrode 760.

In some embodiments, the wearable body 710, the first electrode module 720, the second electrode module 730, and the circuit board 750 may be the same or similar to the wearable body 610, the first electrode module 620, the second electrode module 630, and the circuit board 640, respectively, as shown in FIG. 6, which are not described herein. In some embodiments, the reference electrode 760 may have a split structure, as shown in FIG. 7. For example, the reference electrode 760 may include a plurality of sub-electrodes (e.g., a sub-electrode 761, a sub-electrode 762, a sub-electrode 763). In some embodiments, each of the plurality of sub-electrodes may be connected to the circuit board 750 via a wire. In some embodiments, the plurality of sub-electrodes may be spaced apart and connected by wires (e.g., connected in series). By configuring the reference electrode 760 as a split structure, the signal collection device 700 may use other sub-electrodes as the reference electrodes when one or more of the plurality of sub-electrodes fails (e.g., falls off, short-circuits, etc.), thereby improving the stability of the signal collection device 700. In some embodiments, each sub-electrode of the plurality of sub-electrodes may correspond to one electrode module. For example, the sub-electrode 761 corresponds to the first electrode module 720, the sub-electrode 762 corresponds to the second electrode module 730, and the sub-electrode 763 corresponds to the third electrode module 740. In some embodiments, projections of the plurality of sub-electrodes may at least partially coincide with the first electrode module 720, the second electrode module 730, or the third electrode module 740 along a muscle fiber direction (or a y direction of the wearing body 710). For example, along an x direction, the sub-electrode 761 and the first electrode module 720 may have the same length and position such that the projections of the sub-electrode 761 and the first electrode module 720 along the muscle fiber direction (or the y-direction) may overlap. In some embodiments, when the projections of the plurality of sub-electrodes at least partially coincide with the first electrode module 720, the second electrode module 730, or the third electrode module 740, a length of a region of the wearing body 710 not covered by the electrodes may be increased, thereby reducing an effect of the electrodes on elasticity of the wearing body 710, and increasing an elastic stretching length of the wearing body 710.

In some embodiments, the wearing body 710 may be configured as a leg strap (e.g., a full leg strap, a thigh strap, a calf strap, etc.), a leg sleeve (e.g., a full leg sleeve, a thigh sleeve, a calf sleeve, etc.), or the like. The first electrode module 720 and the second electrode module 730 may be configured to collect EMG signals from different muscles of the leg, respectively. Taking the wearing body 710 as a thigh strap as an example, when the user wears the signal collection device 700, an upper lateral edge 711 may be located at the top (e.g., closer to the root of the thigh) of the wearable body 710, and the first electrode module 720 and the second electrode module 730 may be configured to collect EMG signals from an anterior muscle (e.g., the rectus femoris) and a posterior muscle (e.g., the biceps femoris) of the user's leg, respectively. In some embodiments, since the iliotibial band on the lateral side of the thigh is a non-muscle tissue, it may produce little or no EMG signal. If one or more electrodes are provided on the lateral side of the thigh (e.g., at a same or a similar height as the height of the muscles on the anterior or posterior side of the thigh), an effective EMG signal may not be collected, and even if an EMG signal is collected, the collected EMG signal may be susceptible to interference from adjacent anterior or posterior muscles. However, postural errors or over-fatigue during the user's motion (e.g., running, side leg raises, etc.) may cause iliotibial band syndrome. The iliotibial band therefore needs to be monitored during the monitoring of the user's motion. For example, since the iliotibial band is pulled by the tensor fascia lata of the hip, a condition of the iliotibial band may be determined based on an EMG signal of the tensor fascia lata. The third electrode module 740 may be configured as an additional electrode for collecting an EMG signal of the lateral side of the thigh of the user (e.g., the EMG signal of the tensor fascia lata). In some embodiments, since the tensor fascia lata is higher than the muscles on the anterior or posterior side of the thigh, to collect an effective EMG signal of the lateral side of the thigh while avoiding an influence of the muscles on the anterior or posterior side of the thigh, the third electrode module 740 may be disposed above the first electrode module 720 and the second electrode module 730 in a height direction (or y-direction) of the user.

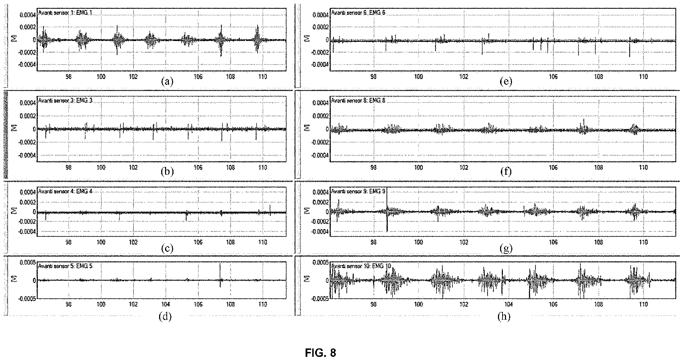

In some embodiments, EMG signals may be collected at a plurality of height positions on the lateral side of the thigh of the user, and a location of the third electrode module 740 may be determined based on the strengths of the EMG signals. FIG. 8 is a schematic diagram illustrating electromyographic (EMG) signals at a plurality of positions on the leg of a user according to some embodiments of the present disclosure. As shown in FIG. 8, signals *a*-*h* are EMG signals of the leg collected at the same moment when the user performs the same action. The signal *a* is the EMG signal of an anterior thigh muscle (e.g., the rectus femoris), and *b-h* are the EMG signals collected at different heights on the lateral side of the thigh, with the collected positions increasing from *b* to *h.* The signal *a* may be used as a reference signal for determining the height of the third electrode module 740. For example, during side leg raises, both the rectus femoris and the vastus tensor fascia lata exert force, and thus when the rectus femoris is exerting force, the signal a may be determined as a reference signal. When the strength of the EMG signal collected at a position on the lateral side is close to the strength of the signal *a*, which indicates that a relatively obvious EMG signal may be obtained at the position, a height of that position may be designated as the height of the third electrode module 740. As shown in FIG. 8, the strength of the signal *g* and the strength of the signal *h* is close to the strength of the signal a, and a position that is higher than the height corresponding to the signal *g* or the signal *h* may be determined as a position of the third electrode module 740. Thus, the position of the third electrode module 740 may be characterized based on the position of the first electrode module 720 or the position of the second electrode module 730. For example, the third electrode module 740 is located above the first electrode module 720 and the second electrode module 730. As another example, based on the height of the first electrode module 720 corresponding to the rectus femoris and the height corresponding to the signal *g* or signal *h,* a ratio of the height difference between the third electrode module 740 and the first electrode module 720 (or the second electrode module 730) to a length of the thigh may be greater than 1/10. By configuring the third electrode module 740, changes in the EMG signals on the lateral side of the thigh may be monitored. For example, the condition of the iliotibial band may be monitored based on the EMG signals of the tensor fasciae lata such that feedback information may be generated when the iliotibial band becomes fatigued, thereby preventing injury to the iliotibial band.

It is to be understood that the signal collection device 600 and the signal collection device 700 illustrated in FIG. 6 and FIG. 7 are only exemplary illustrations and are not limitations of the present disclosure. Based on the principles and methods of the present specification, various modifications may be made to the components of the signal collection device 600 and the signal collection device 700 to adapt to the needs of different scenarios. For example, the signal collection device 600 and the signal collection device 700 may also include one or more other components. As another example, modifications may be made to the location, shape, size, or the like of one or more components of the signal collection device 600 and the signal collection device 700. As yet another example, as exemplified in FIG.6 and FIG.7 with the example of thigh straps, the signal collection device 600 and the signal collection device 700 may also be calf straps, or be part of myoelectric pants, myoelectric suits, or the like.

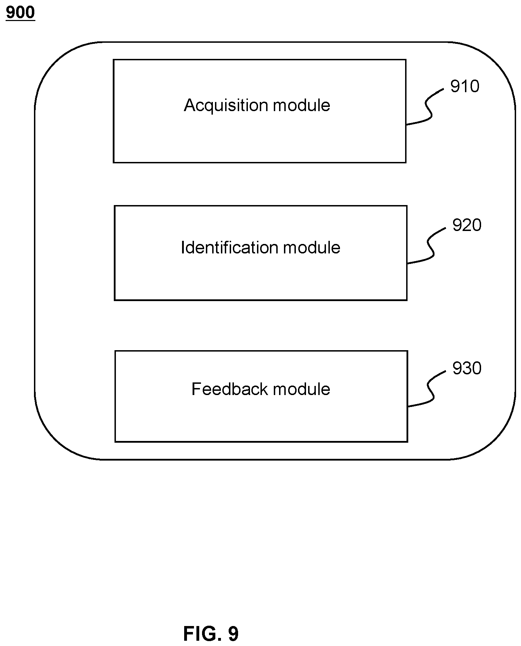

FIG. 9 is a block diagram illustrating an exemplary device for monitoring user's running according to some embodiments of the present disclosure. In some embodiments, a device 900 shown in FIG. 9 for monitoring the user's running (hereinafter referred to as the device 900) may be applied to the system 100 shown in FIG. 1, e.g., configured to the processing device 110 and/or the one or more terminal devices 140 in the form of software and/or hardware for monitoring user's running based on motion signals collected by the signal collection device 150.

In some embodiments, the device 900 may include an acquisition module 910, an identification module 920, and a feedback module 930.

The acquisition module 910 may be configured to collect an EMG signal of a leg muscle of a user when the user is running. The leg muscle may include at least one of a calf muscle, a thigh muscle, a pelvis muscle, a hip muscle, or the like. The EMG signal refers to a bioelectric signal produced during muscle contractions. In some embodiments, the user may use and wear a signal collection device described in some embodiments of the present disclosure (e.g., the signal collection device 150 described in FIG. 1, the signal collection device 200 described in FIG. 2, the signal collection device 600 described in FIG. 6, the signal collection device 700 described in FIG. 7, etc.) during running, so that the EMG signal of the leg muscle of the user during running may be collected by the signal collection device. In some embodiments, the acquisition module 910 may collect EMG signals directly from a signal collection device (e.g., the signal collection device 110). In some embodiments, the EMG signal may be stored in a storage device (e.g., the storage device 130), and the acquisition module 910 may obtain the EMG signal from the storage device.

In some embodiments, the EMG signal may be used to evaluate a motion state (e.g., a running posture, a fatigue state, etc.) of the user during running. Exemplary evaluations of the running posture may include evaluating the user's running skill, balance, stability, or the like during running. In some embodiments, the acquisition module 910 may collect EMG signals from different leg muscles to evaluate different motion states.

The identification module 920 may be configured to identify a plurality of feature values of the EMG signal in a plurality of target time intervals. A target time interval refers to a time period in a running action. In some embodiments, the target time interval may be related to a running cycle of the user. The plurality of feature values may be feature parameters of the EMG signal that reflect the user's motion state. Exemplary feature parameters may include amplitude values, absolute amplitude values, an average amplitude value, a maximum amplitude value, a minimum amplitude value, a medium value frequency, a peak frequency, a total force value, an EMG power, a foot contact time, an average frequency, or the like corresponding to the EMG signal in each of the plurality of target time intervals.

In some embodiments, the identification module 920 may be configured to identify different feature values within different target time intervals for evaluating different motion states. In some embodiments, to identify the plurality of feature values of the EMG signal in the plurality of target time intervals, the identification module 920 may be configured to segment the EMG signals based on a plurality of first time windows and determine first segmented EMG signals in the plurality of first time windows, determine a candidate value of each first segmented EMG signal, and determine, from a plurality of candidate values corresponding to the first segmented EMG signals, candidate values that satisfy a condition as the plurality of feature values. In some embodiments, the duration of each first time window of the plurality of first time windows may be smaller than or equal to the running cycle of the user, and the candidate value of each first segmented EMG signal may include an average amplitude value of the each first segmented EMG signal. In some embodiments, to identify the plurality of feature values of the EMG signals in the plurality of target time intervals, the identification module 920 may further be configured to segment the EMG signal based on a plurality of second time windows and determine second segmented EMG signals in the plurality of second time windows, and determine the plurality of feature values based on candidate values of the second segmented EMG signal in each second time window. In some embodiments, the duration of each second time window may be larger than the duration of each first time window, and the feature value may be a maximum, minimum, intermediate, average, weighted average, or weighted average, etc., of the candidate values of each second segmented EMG signal. In some embodiments, the candidate value of each first segmented EMG signal may include an absolute amplitude value of the first segmented EMG signal, and for each second time window, the feature value may include at least one of a maximum value, a minimum value, an intermediate value, an average value, and a weighted average value of the candidate values of the second segmented EMG signal in the second time window. In some embodiments, the candidate value of each first segmented EMG signal may include an average frequency of the first segmented EMG signal, and for each second time window, the feature value may include at least one of a maximum value, a minimum value, an intermediate value, an average value, and a weighted average value of the candidate values of the second segmented EMG signal in the second time window.

In some embodiments, to identify the plurality of feature values of the EMG signal in the plurality of target time intervals, the identification module 920 may be further configured to segment the EMG signal based on a plurality of third time windows, determine third segmented EMG signals in the plurality of third time windows, and determine a feature value for each segmented EMG signal in the third time window.

The feedback module 930 may be configured to generate running feedback information in response to that the plurality of feature values satisfy a preset condition. In some embodiments, the preset condition may be a critical condition for determining the running feedback information. For example, the preset condition may include a feature parameter that indicates a change in the plurality of feature values being greater than a preset threshold. As another example, the plurality of feature values may include a maximal value, and the preset condition may include the plurality of feature values including a feature value greater than the maximal value. As yet another example, the EMG signal may include a plurality of EMG signals corresponding to a plurality of muscles of a leg of the user, the plurality of feature values may include amplitude ratios of EMG signals of at least two muscles of the plurality of muscles during a same running action, and the preset condition may include a difference between at least two feature values being greater than a difference threshold. In some embodiments, the feedback module 930 may be configured to evaluate the user's motion state based on different preset conditions and generate running feedback information based on evaluation results. Exemplary running feedback information may include at least one of: a fatigue reminder, a running workout amount recommendation, a target muscle strength training recommendation, or the like. In some embodiments, the running feedback information may be delivered to the user in the form of voice, text, images, etc., based on the signal collection device or a terminal device (e.g., a headset, a pair of smart glasses, a cell phone, etc.).

It should be understood that the device 900 and its modules for monitoring user's running, as illustrated in FIG. 9, may be implemented in a variety of manners. For example, in some embodiments, the device and its modules may be implemented by hardware, software, or a combination of software and hardware. In this regard, the hardware portion may be implemented utilizing dedicated logic; the software portion may be stored in memory and executed by an appropriate instruction execution system, such as a microprocessor or dedicated design hardware. Those skilled in the art should understand that the methods and systems described above may be implemented using computer-executable instructions and/or included in processor control code, such as provided on carrier media such as disks, CDs or DVD-ROMs, programmable memory such as read-only memories (firmware), or data carriers such as optical or electronic signal carriers. The device and its modules of the present disclosure may be realized not only with hardware circuits such as ultra-large scale integrated circuits or gate arrays, semiconductors such as logic chips, transistors, etc., or programmable hardware devices such as field-programmable gate arrays, programmable logic devices, etc., but also with software executed, for example, by processors of various types, or with a combination of the above hardware circuits and software (e.g., firmware).

It should be noted that the above description of the device 900 for monitoring user's running and its modules is provided only for descriptive convenience, and does not limit the present disclosure to the scope of the cited embodiments. It is to be understood that for those skilled in the art, after understanding the principle of the system, it is possible to arbitrarily combine the individual modules or form a sub-system to be connected to the other modules without deviating from this principle. In some embodiments, the acquisition module 910, the identification module 920, and the feedback module 930 disclosed in FIG. 9 may be different modules in a system, or a single module realizing the functions of two or more modules described above. For example, the individual modules may share a common storage module, and the individual modules may each have a respective storage module. Such modifications are within the protection scope of the present disclosure.

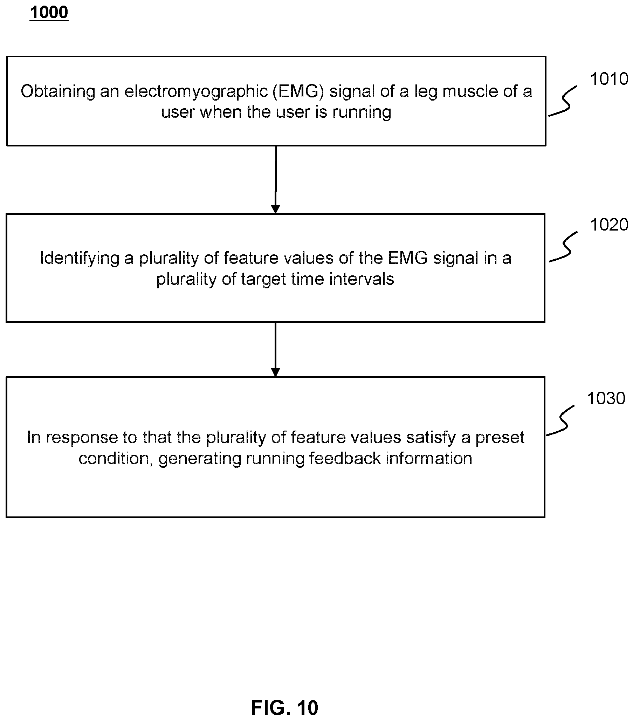

FIG. 10 is a flowchart illustrating an exemplary process of monitoring user's running according to some embodiments of the present disclosure. In some embodiments, process 1000 may be performed by processing logic that may include hardware (e.g., circuitry, specialized logic, programmable logic, microcode, etc.), software (running on a processing device to execute instructions simulated by the hardware), etc., or any combination thereof. In some embodiments, one or more of the operations in the process 1000 for monitoring the user's running shown in FIG. 10 may be implemented by the processing device 110 and/or the one or more terminal devices 140 shown in FIG. 1. For example, process 1000 may be stored in the form of instructions in the storage device 130 and called and/or executed by the processing device 110 and/or the one or more terminal devices 140. The following takes the process 1000 performed by the processing device 110 as an example. As shown in FIG. 10, process 1000 may include the following operations.

In 1010, an EMG signal of the leg muscle of a user may be obtained when the user is running. In some embodiments, operation 1010 may be performed by the acquisition module 910.

During running, muscles in various parts (e.g., legs, hips, pelvis, arms, etc.) of the user's body exert force and generate EMG signals to complete the running motion. The EMG signals refer to bioelectric signals produced during muscle contractions. In some embodiments, the user may use or wear a signal collection device described in the present disclosure (e.g., the signal collection device 150 described in FIG. 1, the signal collection device 200 described in FIG. 2, the signal collection device 600 described in FIG. 6, the signal collection device 700 described in FIG. 7, etc.) during running to collect the EMG signal from the leg muscle. In some embodiments, the processing device 110 may directly collect the EMG signal using the signal collection device (e.g., the signal collection device 110). In some embodiments, the EMG signal may be stored in a storage device (e.g., the storage device 130), and the processing device 110 may retrieve the EMG signal from the storage device. The present disclosure describes a method for monitoring a user's running by taking a leg muscle and related parts (e.g., hips, pelvis, etc.) as an example. It should be noted that the method provided in the present disclosure may monitor the user's running or other motions based on EMG signals of other parts of the user's body or other signals (e.g., posture signals, mechanical signals, electrocardiographic signals, respiratory signals, sweat signals, etc.).

The leg muscle may refer to a single muscle or a group of muscles in the leg that the user uses when running. In some embodiments, the leg muscle may include a calf muscle and/or a thigh muscle. Exemplary calf muscles may include tibialis anterior, extensor digitorum longus, extensor hallucis longus, peroneus longus, peroneus brevis, gastrocnemius, soleus, plantaris, popliteus, flexor digitorum longus, flexor digitorum longus, tibialis posterior, or the like. Exemplary thigh muscles may include quadriceps femoris, sartorius, gracilis, adductor longus, adductor magnus, pectineus, biceps femoris, semitendinosus, semimembranosus, or the like. In some embodiments, the movement of the legs of the user while running may also involve other parts of muscles or muscle groups associated with the legs. In such cases, the processing device 110 may also obtain EMG signals generated by the muscles of the parts associated with the legs for running monitoring. For example, when the user is running, the parts associated with the legs may include the pelvis, hips, or the like. Exemplary hip muscles may include the tensor fasciae lata, gluteus medius, gluteus maximus, gluteus minimus, piriformis, superior gemellus, obturator internus, inferior gemellus, quadratus femoris, obturator externus, or the like; and exemplary pelvic muscles may include the iliopsoas, etc.

In some embodiments, the EMG signal may be used to evaluate a motion state (e.g., a running posture, a fatigue state, etc.) of the user during running. Exemplary evaluation of the running posture may include evaluating the user's running skill, balance, stability, or the like during running. The running skill refers to whether the user's leg muscles are exerting force correctly. For example, whether the leg muscles are exerting force correctly may be determined based on a force exertion status of the user's leg muscles (e.g., whether the muscles are exerting force, an order of exertion, an intensity of exertion, etc.). The balance refers to the symmetry of the user's leg muscles during running, for example, whether the EMG signals of the muscles in the symmetrical parts of the left leg and the right leg are the same or similar. The stability refers to fluctuations in the EMG signal during different running cycles. Merely by way of example, during the user's running, the leg muscles move in a cyclical manner, wherein when the leg is in the air, some of the leg muscles (e.g., the calf muscles) are relaxed, and an amplitude of the EMG signal may be a minimum value within a cycle; and when the leg (i.e., the foot) touches the ground, some of the leg muscles tighten, and the amplitude of the EMG signal may be a maximum value within the cycle. Therefore, the force exertion status of the leg muscles may be determined based on the amplitude of the EMG signal, thereby determining the state of the leg muscles. As another example, as the running time increases, some of the leg muscles gradually enter a fatigue state, and in the fatigue state, the leg muscles may also tighten when the leg is in the air due to exertion. In such cases, whether the user's leg is in the fatigue state may be determined based on a trend of changes in the EMG signal in different running cycles. As yet another example, the EMG signal may include a plurality of EMG signals corresponding to a plurality of muscles of the user's legs. The user's running posture or the fatigue state may be evaluated based on a relationship (e.g., a numerical relationship, etc.) between the plurality of EMG signals. In some embodiments, the processing device 110 may collect EMG signals from different leg muscles to evaluate different motion states.

In 1020, a plurality of feature values of the EMG signal in a plurality of target time intervals may be identified. In some embodiments, operation 1020 may be performed by the identification module 920.

A target time interval refers to a time period in a running action. In some embodiments, the target time interval may be related to a running cycle of the user. For example, the target time interval may be greater than the running cycle of the user. As another example, the target time interval may be less than or equal to the running cycle of the user. As yet another example, the running cycle of the user may be divided into a plurality of phases (e.g., foot contacting the ground, foot push-off, leg folding, leg swing forward, etc.), and the target time interval may correspond to one or more of the plurality of phases. In some embodiments, the EMG signals may include a plurality of EMG signals corresponding to a plurality of leg muscles of the user. A target time interval may be the same time period corresponding to the plurality of EMG signals. In some embodiments, the processing device 110 may segment the EMG signal based on a plurality of time windows and determine an average amplitude of each segmented EMG signal as a feature value of the segmented EMG signal. In some embodiments, the processing device 110 may segment the EMG signal based on a plurality of first time windows and determine a candidate value of each first segmented EMG signal. Further, the processing device 110 may segment the EMG signal based on a plurality of second windows and determine the plurality of feature values based on candidate values of the second segmented EMG signal in each second time window. In some embodiments, the processing device 110 may directly designate a preset time period in a single running cycle as a target time interval to obtain the plurality of feature values of the EMG signal.

The plurality of feature values refer to feature parameters in the EMG signal that characterize a motion state of the user. Exemplary feature parameters may include amplitude values, absolute amplitude values, an average amplitude value, a maximum amplitude value, a minimum amplitude value, a medium value frequency, a peak frequency, a total force value, an EMG power, a foot contact time, or an average frequency corresponding to the EMG signal in each of the plurality of target time intervals. In some embodiments, when the user's motion state changes (e.g., running posture changes, entering a fatigue state, etc.), the force exertion status of the leg muscles changes, and correspondingly, the feature parameters reflecting the user's motion state may change. In such cases, the motion state of the user may be evaluated based on the feature parameters. For example, a total force of the leg muscles in the fatigue state is greater than a total force of the leg muscles in a non-fatigue state. The total force refers to a sum of amplitudes (e.g., the sum of absolute amplitude values, the sum of multiple powers of amplitudes, the sum of multiple times of amplitudes, etc.) of the EMG signal in a preset time period (e.g., a running cycle, a time period in which the foot contacts the ground, a time period in which the leg is in the air, etc.). As another example, an EMG power of the leg muscles in the fatigue state may be greater than an EMG power of the leg muscles in the non-fatigue state. The EMG power refers to a sum of amplitudes of the EMG signal per unit of time. For example, in the non-fatigue state, a portion of the leg muscles are relaxed when the leg is in the air, and the corresponding amplitude of the EMG signal of the portion of the leg muscles may be a minimum value in a running cycle; and in the fatigue state, the portion of the leg muscles is in a tightened state when the leg is in the air, for example, the amplitude of the EMG signal of the portion of the leg muscles increases when the leg is in the air. As another example, in the non-fatigue state, a portion of the leg muscles tightens when the foot contacts the ground, and the corresponding amplitude of the EMG signal may be a maximum value in a running cycle; and in the fatigue state, a muscle strength of the portion of the leg muscles abnormally increases when the foot contacts the ground, for example, the amplitude of the EMG signal increases at a speed greater than a speed threshold. As yet another example, since a portion of the leg muscles tighten when the foot contacts the ground, the amplitude of the corresponding EMG signal may be greater than a preset amplitude threshold, whereby the time period in which the foot contacts the ground may be determined. In some embodiments, the time period in which the foot contacts the ground in the fatigue state may be longer than the time period in which the foot contacts the ground in the non-fatigue state. As still another example, an average frequency of the leg muscles decreases as the leg muscles move from the non-fatigue state to the fatigue state. The average frequency refers to a ratio of a weighted sum of the amplitude of the EMG signal at each frequency to a total sum of the amplitude of the EMG signal at the each frequency, wherein the weighted sum may include the multiplication of the amplitude at each frequency by a corresponding frequency, the multiple powers of the amplitude at each frequency, the multiple values of the amplitude at each frequency, etc.

In some embodiments, the plurality of target time intervals and/or the plurality of feature values corresponding to the EMG signal to be evaluated may be different for different motion states. In such cases, the processing device 110 may identify different feature values in different target time intervals for evaluating different motion states. For example, the processing device 110 may identify the amplitudes of the EMG signals in any same time interval as the feature values when evaluating the balance of the user's running posture. As another example, in determining a fatigue state during the user's running, for each segmented EMG signal in each of plurality of time windows, the processing device 110 may identify an average amplitude of the segmented EMG signal as a feature value, wherein each time window is less than or equal to the running cycle. As yet another example, when evaluating the running skill in the user's running, the processing device 110 may identify the amplitude of the EMG signal of a preset leg muscle (e.g., the biceps femoris) during a preset time period (e.g., a leg folding phase) in the running cycle as a feature value. In some embodiments, the processing device 110 may identify a plurality of feature values in a plurality of target time intervals based on a preset motion state to be evaluated. For example, the user may select the motion state to be evaluated via a terminal device (e.g., the one or more terminal devices 140), and the processing device 110 may determine a feature value based on the motion state selected by the user. In some embodiments, the processing device 110 may identify the plurality of feature values in the plurality of target time intervals based on all motion states tobe evaluated to evaluate each motion state.

In 1030, in response to that the plurality of feature values satisfy a preset condition, running feedback information may be generated. In some embodiments, operation 1030 may be performed by the feedback module 930.

In some embodiments, the preset condition refers to a critical condition for determining the running feedback information. In some embodiments, the processing device 110 may evaluate the user's motion state based on different preset conditions and generate running feedback information based on evaluation results. Exemplary running feedback information may include at least one of: a fatigue reminder, a running workout amount recommendation, a target muscle strength training recommendation, or the like. In some embodiments, the running feedback information may be delivered to the user in the form of voice, text, images, etc., based on the signal collection device or a terminal device (e.g., a headset, a pair of smart glasses, a cell phone, etc.).

Taking the fatigue reminder as an example, with the increase of running time, the user's leg muscle gradually enters into the fatigue state. When the muscle is over-fatigued, continuing the running action may cause an injury, such as muscle cramps, rhabdomyolysis, a joint injury, etc., since the muscles can not provide enough linking force for the joints. Therefore, whether the user's leg muscle is over-fatigued may be determined based on the preset condition and a corresponding fatigue reminder may be generated when the leg muscle is over-fatigued. Merely by way of example, the preset condition corresponding to over-fatigue may include a feature parameter that indicates a change in the plurality of feature values being greater than a preset threshold. For example, a curve of the plurality of feature values over time may be obtained, and a slope of the curve may be designated as the feature parameter indicating the change in the plurality of feature values. When the slope of the curve is greater than a preset slope threshold, it may be determined that the user's leg muscle is in the fatigue state. As another example, one or more differences between a later feature value and a prior feature value may be obtained based on a temporal order, and the differences may be designated as the feature parameters indicating the change in the plurality of feature values. When the difference is greater than a preset difference threshold, it may be determined that the user's leg muscle is in the fatigue state. As yet another example, the plurality of feature values may include a maximal value, and the preset condition corresponding to over-fatigue may include that the plurality of feature values include a feature value greater than the maximal value. As still another example, the preset condition corresponding to the over-fatigue may include that the plurality of feature values include a feature value greater than a reference threshold. The reference threshold may be determined based on historical EMG signals from historical users (e.g., the current user, users other than the current user, etc.). For example, the reference threshold may be a maximum value of the plurality of feature values of the historical EMG signals. As another example, the reference threshold may be 90%, 80%, 70%, or the like of the maximum value of the plurality of feature values.

In some embodiments, when the leg muscle is in the over-fatigue state, continuing the running for a certain duration (e.g., 1 hour, 40 minutes, 20 minutes, 5 minutes, 30 seconds, etc.) or a certain distance (e.g., 5 km, 3 km, 500 m, etc.) may cause an injury. Therefore, a running workout amount recommendation may be generated after determining that the user is in the over-fatigue state. For example, if continuing the running for 40 minutes after the muscle is in the over-fatigue state may cause an injury, the running workout amount recommendation may be to continue running for 20 minutes, 10 minutes, or to stop immediately after the muscle enters the over-fatigue state. In some embodiments, the running workout amount recommendation may also be generated based on historical EMG signals. For example, the running workout amount recommendation may be generated based on a historical running workout amount (e.g., an average or minimum of running workout amount from a plurality of historical users) corresponding to muscle over-fatigue determined from historical EMG signals. As another example, the historical running workout amount may be combined with the current EMG signal to generate the running workout amount recommendation for the current user.

Taking a target muscle strength training recommendation as an example, during the running process, a plurality of leg muscles need to work together to complete the running. When some of the plurality of leg muscles are weak, there may be insufficient muscle force, insufficient stability or balance, and easy fatigue of the muscles, which may cause problems such as difficulty in increasing the running speed, and easy injury to the muscles or other parts of the body (e.g., the knees). Therefore, based on a preset condition, whether there is a target muscle that needs to be trained may be determined and the target muscle strength training recommendation may be generated. For example, the plurality of feature values may include an amplitude value (e.g., a maximum amplitude value, a minimum amplitude value, an average amplitude value, etc., during a running cycle) of the EMG signal, and the corresponding preset condition may include that the amplitude increases at a speed greater than a speed threshold. When the amplitude of the EMG signal of a current muscle increases at a speed greater than the speed threshold, which indicates that a strength of the current muscle is abnormally increased, the current muscle may be determined as the target muscle, and the target muscle strength training recommendation may be generated. As another example, the plurality of feature values may include a feature value corresponding to the fatigue state, and the corresponding preset condition may include that a running workout amount (e.g., running duration or distance) when the current muscle is in a fatigue state is less than a workout amount threshold. When the running workout amount when the current muscle is in a fatigue state is less than the workout amount threshold, the current muscle may be determined as the target muscle and the target muscle strength training recommendation may be generated. As yet another example, the plurality of feature values may include a feature value corresponding to the stability, and the corresponding preset condition may include that a time when a stability problem occurs is less than a preset time threshold. In some embodiments, the target muscle strength training recommendation may also be generated based on a plurality of EMG signals corresponding to a plurality of leg muscles. For example, the plurality of feature values may include an amplitude value of a first EMG signal of a first muscle and an amplitude value of a second EMG signal of a second muscle (e.g., the amplitude values, the absolute amplitude values, the maximum amplitude values, the minimum amplitude values, the average amplitude values, etc., of the two EMG signals at the same moment), and the corresponding preset condition may include that a ratio of the amplitude increase speeds of the two EMG signals is greater than a ratio threshold. When the ratio of the amplitude increase speed of the first EMG signal to that of the second EMG signal is greater than the ratio threshold, which indicates that the strength of the first muscle is abnormally elevated relative to the strength of the second muscle, the first muscle may be determined as the target muscle, and the target muscle strength training recommendation may be generated. In some embodiments, the target muscle strength training recommendation may include a target muscle recommended for training, a recommended workout amount, a training technique, etc., or any combination thereof. For example, the preset condition may include one or more grades, and each grade may correspond to a different recommended workout amount and/or training technique. The processing device 110 may determine the grade of the preset condition satisfied by the plurality of feature values and determine the recommended workout amount and/or training technique based on the grade.

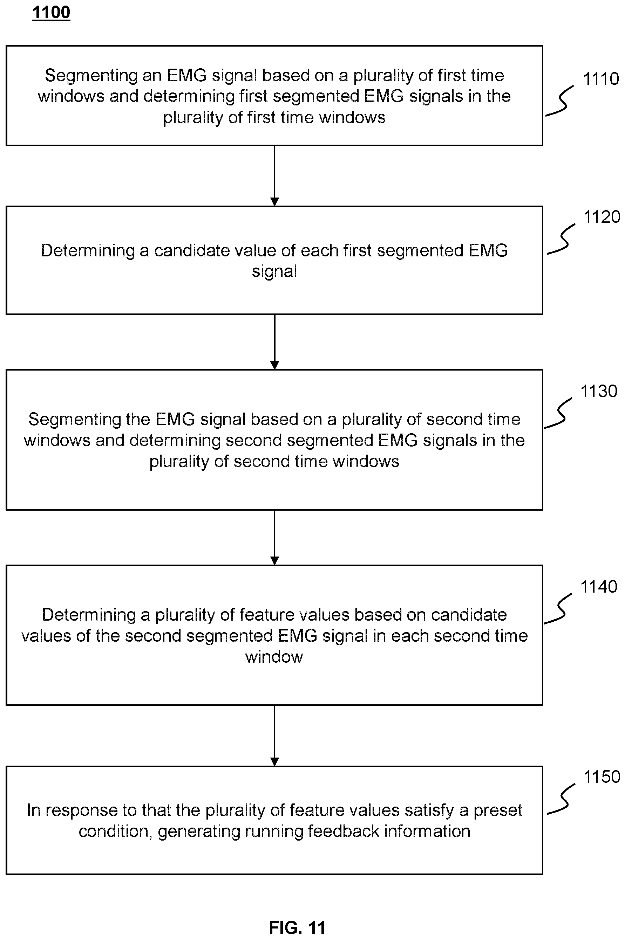

It should be noted that the above process for generating the running feedback information based on the preset condition is only an exemplary illustration, and in some embodiments, other running feedback information may be generated based on other preset conditions. Merely by way of example, the preset condition may also include conditions related to the user's running posture (e.g., running skill, balance, stability, etc.). For example, an exemplary running skill may include that the biceps femoris needs to actively exert force during the leg folding phase of a running cycle, and correspondingly, a relatively obvious EMG signal may be generated during the leg folding phase. Therefore, an amplitude value (e.g., an absolute amplitude value) of the EMG signal of the biceps femoris muscle during the leg folding phase may be determined as a feature value, and a preset condition associated with the running skill may include that the amplitude of the EMG signal during the leg folding phase is less than a preset amplitude threshold. When the amplitude of the EMG signals during the leg folding phase is less than a preset amplitude threshold, the biceps femoris may be determined to be under-exerting during the phase and corresponding running feedback information may be generated. As another example, the exemplary running skill may further include that the biceps femoris does not exert force during a leg swing forward phase of a running cycle, and correspondingly, the biceps femoris does not generate an obvious EMG signal during the leg swing forward phase. In such cases, the amplitude value (e.g., the absolute amplitude value) of the EMG signal of the biceps femoris during the leg swing forward phase may be determined as a feature value, and a preset condition associated with the running skill may include that the amplitude value of the EMG signal during the leg swing forward phase is greater than a preset amplitude threshold. When the amplitude value of the EMG signal in the leg swing forward phase is greater than the preset amplitude threshold, the biceps femoris may be determined to be exerting excessive force in the phase, and corresponding running feedback information may be generated. As another example, a preset condition related to symmetry in the running posture may include that an amplitude difference between the EMG signals of the muscles of symmetrical parts of the left leg and the right leg during the same time period is greater than a difference threshold. In response to that the amplitude difference is greater than the difference threshold, it may be determined that there is an asymmetry problem in the muscles of the symmetrical parts and corresponding running feedback information may be generated. As yet another example, a preset condition related to the stability in the running posture may include a fluctuation in the amplitude value of EMG signals of the current muscle during the same phase in different running cycles (e.g., the amplitude difference of the amplitudes in different running cycles is less than a difference threshold). In response to the fluctuation in the amplitude value of the EMG signals of the current muscle during the same phase in different running cycles, it may be determined that a stability problem occurs in the current movement of the muscle, and corresponding running feedback information may be generated. As still another example, a preset condition related to the stability in the running posture may include a fluctuation in an amplitude ratio of EMG signals of two muscles during the same phase in different running cycles (e.g., a difference between the amplitude ratios in different running cycles is less than the difference threshold). More descriptions of identifying the plurality of feature values of the EMG signal and generating the running feedback information may be found elsewhere in the present disclosure (e.g., FIGS. 11-15 and related descriptions thereof) and will not be repeated here.

According to the method of monitoring the user's running described in some embodiments of the present disclosure, real-time monitoring of the leg muscles during the user's running can be achieved, and timely running feedback information can be generated when an abnormal motion state is likely to occur, thereby preventing the user from getting injured during running. In addition, the method described in the present disclosure can also achieve comprehensive monitoring of the user's running process, which facilitates the evaluation of the motion state of the user's leg muscles during the running process, thereby generating appropriate running feedback information (e.g., the running workout amount recommendation, the target muscle strength training recommendation, etc.) based on the motion state, improving the user experience.

It should be noted that the foregoing description of process 1000 is intended to be exemplary and illustrative only, and does not limit the scope of the present disclosure. For those skilled in the art, various modifications and variations may be made to process 1000 under the guidance of the present disclosure. However, these modifications and variations remain within the scope of the present disclosure. For example, process 1000 is illustrated with the EMG signals of the leg muscles as an example, and in some embodiments, monitoring of the user's running or other motions may be performed based on EMG signals of other parts of the body or other signals (e.g., posture signals, mechanics signals, ECG signals, respiration signals, sweat signals, etc.). For example, EMG signals from the user's waist, arms, back, etc., may be obtained and combined with the EMG signals of the leg muscles for monitoring the user's running. As another example, running data (e.g., stride frequency, stride length, speed, acceleration, explosive power, ground contact time, in air time, in air height, left-right balance, etc.) may be obtained based on inertial sensors and combined with the EMG signals for monitoring the user's running.

FIG. 11 is a flowchart illustrating an exemplary process for identifying a plurality of feature values of an EMG signal and generating running feedback information according to some embodiments of the present disclosure. In some embodiments, process 1100 may be performed by processing logic that may include hardware (e.g., circuitry, specialized logic, programmable logic, microcode, etc.), software (running on a processing device to execute instructions simulated by the hardware), etc., or any combination thereof. In some embodiments, one or more of the operations in process 1100 shown in FIG. 11 may be performed by the processing device 110 and/or the one or more terminal devices 140 shown in FIG. 1. For example, process 1100 may be stored in the form of instructions in the storage device 130 and called and/or executed by the processing device 110 and/or the one or more terminal devices 140. In some embodiments, operation 1020 and operation 1030 of process 1000 for monitoring user's running may be implemented by process 1100. In some embodiments, process 1100 may be performed by the identification module 920 and the feedback module 930. The following takes the process 1100 performed by the processing device 110 as an example. As shown in FIG. 11, process 1100 may include the following operations.

In 1110, an EMG signal may be segmented based on a plurality of first time windows and first segmented EMG signals in the plurality of first time windows may be determined.

In 1120, a candidate value of each first segmented EMG signal may be determined.

In some embodiments, the processing device 110 may segment the EMG signal based on a plurality of first time windows, determine first segmented EMG signals in the plurality of first time windows, and determine a candidate value of each first segmented EMG signal.

In some embodiments, the candidate value of each first segmented EMG signal may include one of absolute amplitude values, an average amplitude value (or average absolute amplitude value), a maximum amplitude value, a minimum amplitude value, a medium value frequency, a peak frequency, a total force value, an EMG power, a foot contact time, an average frequency, or the like, corresponding to the segmented EMG signal. Merely by way of example, the candidate value of each segmented EMG signal may include an average amplitude value of the segmented EMG signal. For example, the processing device 110 may determine the absolute amplitude values of each segmented EMG signal, and then determine an average of the absolute amplitude values of the first segmented EMG signal in each first time window as the candidate value. By determining the average of the absolute amplitude values of each first segmented EMG signal, smoothing of the EMG signal may be achieved through the plurality of first time windows, thereby reducing the redundancy of raw EMG signals and facilitating subsequent extraction of the feature values. In some embodiments, the candidate value in each first time window may be used to reflect a feature of the EMG signal in the first time window. For example, a magnitude of the candidate value in each first time window may reflect the magnitude of the EMG signal in the first time window, and thus reflect a force exertion status of the leg muscle in the first time window. In some embodiments, when the user's motion state changes (e.g., running posture changes, enters a fatigue state, etc.), the force exertion status of the leg muscles changes, and the user's motion state may be evaluated based on a change in the EMG signals during a same time period in different running cycles. In some embodiments, the duration of each first time window of the plurality of first time windows is smaller than or equal to a running cycle of the user, such that a candidate value may be determined within a running cycle to reflect the feature of the EMG signal within the single running cycle. In some embodiments, the duration of each first time window of the plurality of first time windows may also be determined based on a time period corresponding to the EMG signal to be evaluated. For example, when the EMG signals to be evaluated are EMG signals of the leg when the leg is in the air, a difference between the duration of the first time window and the duration of the time period in which the leg is in the air (also referred to as in air time of the leg) may be less than a preset time difference threshold (e.g., 0.05 s, 0.03 s, 0.01 s, etc.), or a ratio of the duration of the first time window to the in air time of the leg may be within a preset ratio range (e.g., a range of 1/5-3, a range of 1/4-2, a range of 1/3-1 range, etc.), so that the determined candidate value may reflect the force exertion of the leg muscles during the in air time of the leg, which may smooth the EMG signal while retaining the feature of the EMG signal in the first time window for the evaluation of the motion state. Merely by way of example, if the user's motion state is evaluated based on the EMG signal during a foot contact time, and the foot contact time is 0.04 s, the first time window may be in a range of 0.01 s-0.08 s. If the user's motion state is evaluated based on the EMG signal during the in air time of the leg and the in air time of the leg is 0.05 s, the first time window may be in a range of 0.01 s-0.1 s.

In 1130, the EMG signal may be segmented based on a plurality of second time windows and second segmented EMG signals in the plurality of second time windows may be determined.

In some embodiments, the processing device 110 may segment the EMG signal based on a plurality of second time windows, and determine second segmented EMG signals in the plurality of second time windows. In some embodiments, the duration of each second time window may be larger than the duration of each first time window such that each second segmented EMG signal may include a plurality of candidate values. Further, the processing device 110 may select one or more candidate values from the plurality of candidate values in the second time window as the feature value(s) in the target time interval.

In 1140, the plurality of feature values may be determined based on the candidate values of the segmented second EMG signal in each second time window. In some embodiments, for each second segmented EMG signal in each second time window, the processing device 110 may determine candidate values that satisfy a condition as the plurality of feature values, thereby identifying the plurality of feature values of the EMG signal in the plurality of target time intervals.

In some embodiments, to determine the plurality of feature values based on the candidate values of the segmented second EMG signal, for each second time window, the processing device 110 may determine at least one of a maximum value, a minimum value, an intermediate value, an average value, and a weighted average value of the candidate values of the second segmented EMG signal in the second time window as the plurality of feature values. In some embodiments, the plurality of feature values may be determined based on the plurality of second time windows by selecting feature values from a plurality of candidate values that may represent the features of the muscle during a time period (e.g., leg in the air, foot contact, etc.) of the running cycle. Further, by determining the plurality of feature values based on the plurality of second time windows, a trend of changes in leg muscles over different running cycles may be identified, which allows for monitoring the user's running based on the trend of changes in the feature values). For example, in the non-fatigue state, a leg muscle (e.g., at least one of the quadriceps femoris, sartorius, gracilis, adductor longus, adductor magnus, pectineus, biceps femoris, semitendinosus, semimembranosus, etc. of the thigh muscles, or at least one of the tibialis anterior, extensor digitorum longus, extensor hallucis longus, peroneus longus, peroneus brevis, gastrocnemius, soleus, plantaris, popliteus, flexor digitorum, flexor hallucis brevis, tibialis posterior, etc. of the calf muscles) tightens when the foot contacts the ground, and the amplitude of the corresponding EMG signal may be the maximum value in a running cycle. However, in the fatigue state, the strength of the leg muscle abnormally increases compared to that in the non-fatigued state. Therefore, an evaluation of the motion state may be performed based on the EMG signal of the leg muscle when the foot contacts the ground. Correspondingly, a maximum value of the candidate values of each second segmented EMG signal in the second time window may be selected as the plurality of feature values, which may reflect the force exertion of the leg muscle when the foot contacts the ground. As another example, in the non-fatigue state, a leg muscle (e.g., at least one of the quadriceps femoris, sartorius, gracilis, adductor longus, adductor magnus, pectineus, biceps femoris, semitendinosus, semimembranosus, etc. of the thigh muscles, or at least one of the tibialis anterior, extensor digitorum longus, extensor hallucis longus, peroneus longus, peroneus brevis, gastrocnemius, soleus, plantaris, popliteus, flexor digitorum, flexor hallucis brevis, tibialis posterior, etc. of the calf muscles) is relaxed when the leg is in the air, and the amplitude of the corresponding EMG signal may be the minimum within a running cycle. However, in the fatigued state, the leg muscle remains tightened when the leg is in the air. Therefore, an evaluation of the motion state may be performed based on the EMG signal of the leg muscle when the leg is in the air. correspondingly, a minimum value of the candidate values of each second segmented EMG signal in the second time window may be determined as the plurality of feature values, which may reflect the force exertion of the leg muscle when the leg is in the air. As another example, compared to the non-fatigued state, the strength of the leg muscle in the fatigue state abnormally increases during all phases of the running cycle. Therefore, a median value, an average value, a weighted average value, etc., of each second segmented EMG signal within the second time window may be determined as the plurality of feature values, which may reflect an overall force exertion of the leg muscle during various phases of the running cycle.

In 1150, in response to that the plurality of feature values satisfy a preset condition, running feedback information may be generated.

In some embodiments, the processing device 110 may evaluate whether the leg muscles are in an abnormal motion state based on a change in the EMG signal, and generate running feedback information when the abnormal motion state occurs. For example, in the fatigue state, the force exertion of the leg muscle changes, and when the leg muscle is overly fatigue, the force exertion changes more significantly. Therefore, in some embodiments, the preset condition may include that a feature parameter indicating a change in the plurality of feature values is greater than a preset threshold. The feature parameter indicating the change in the plurality of feature values may include a slope of a curve corresponding to the plurality of feature values, a difference between different feature values, or the like. For example, a curve corresponding to the plurality of feature values over time may be obtained, and when the slope of the curves is greater than a preset slope threshold, which indicates that the EMG signal corresponding to the plurality of feature values changes significantly (e.g., an abnormal increase of the muscle strength when the foot contacts the ground, an abnormal tightening of the leg muscle when the leg is in the air, etc.), it may be determined that the user's leg muscle is in the over-fatigue state. As another example, one or more differences (e.g., one or more difference values of one or more pairs of neighboring feature values, an average value of the one or more difference values, etc.) between a later feature value and a prior feature value may be obtained based on a temporal order. When the one or more difference values are greater than a preset difference threshold, which indicates that the EMG signal corresponding to the feature value changes significantly, it may be determined that the user's leg muscle is in the over-fatigue state. In some embodiments, when it is determined that the user's leg muscle is in the over-fatigue state, the processing device 110 may generate fatigue feedback information. The fatigue feedback information may be delivered to the user in the form of voice, text, images, etc., based on the signal collection device or a terminal device (e.g., a headset, a pair of smart glasses, a cell phone, etc.).

In some embodiments, when the leg muscle is in the over-fatigue state, continuing the running for a certain duration (e.g., 1 hour, 40 minutes, 20 minutes, 5 minutes, 30 seconds, etc.) or a certain distance (e.g., 5 km, 3 km, 500 m, etc.) may cause an injury. Therefore, a running workout amount recommendation may be generated after determining that the user is in the fatigue state. For example, if continuing running for 40 minutes after the muscle is in the overfatigue state may cause an injury, the running workout amount recommendation may be to continue running for 20 minutes, 10 minutes, or to stop immediately after the muscle is in the over-fatigue state. In some embodiments, the running workout amount recommendation may also be generated based on historical EMG signals. For example, the running workout amount recommendation may be generated based on a historical running workout amount (e.g., an average or minimum of running workout amount of a plurality of historical users) corresponding to muscle over-fatigue determined from historical EMG signals. As another example, the historical running workout amount may be combined with the current EMG signal to generate the running workout amount recommendations for the current user.

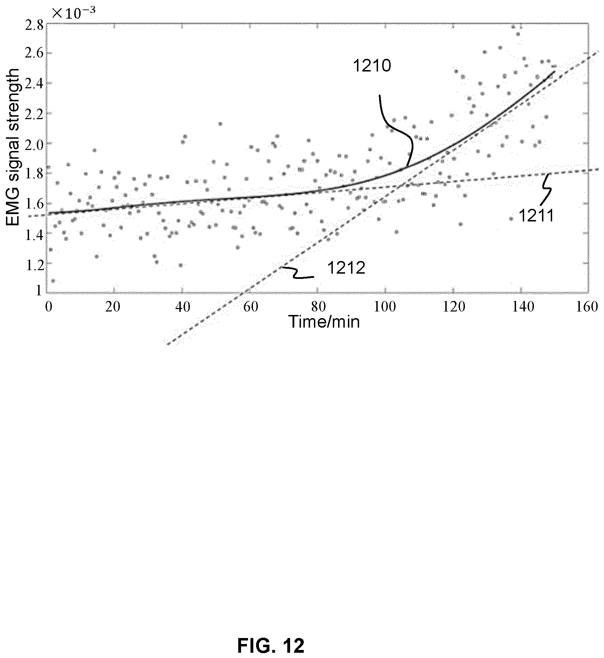

Merely by way of example, FIG.12 is a schematic diagram illustrating a curve showing a change in a plurality of feature values of an EMG signal of a leg muscle of a user over time. The longitudinal coordinate of each coordinate point shown in FIG.12 may represent a feature value of each second segmented EMG signal in a second time window, which may reflect the amount of force generated by the leg muscle. The larger the longitudinal coordinate of each coordinate point, the greater the force generated by the muscle, and the tighter the muscle. For example, the leg muscle may be the gastrocnemius muscle of the user's calf, and the longitudinal coordinate may be one of a maximum value, a minimum value, a medium value, an average value, a weighted average, etc., of the candidate values in the second time window. A curve 1210 is a fitting curve generated based on the plurality of feature values. When the longitudinal coordinate increases abnormally (e.g., a slope of curve 1210 is greater than a preset slope threshold), it may indicate that the muscle exerts force abnormally due to over-fatigue. As shown in FIG. 12, the curve 1210 has a relatively small slope 1211 until 100 minutes. After 100 minutes, the longitudinal coordinates of the coordinate points increase rapidly, and the curve 1210 has a relatively large slope 1212, indicating that the user's leg muscle exerts force abnormally after 100 minutes, and it may be determined that the leg muscle is in the over-fatigue state and fatigue feedback information may be generated. It should be noted that the feature values and the corresponding curve shown in FIG. 12 are only exemplary illustrations, and in some embodiments, when the muscle exerts force abnormally due to over-fatigue, the plurality of feature values corresponding to the EMG signal may also show other trends of changes. For example, the plurality of feature values corresponding to the EMG signal may be abnormally reduced when the muscle is in the over-fatigue state, and an absolute value of a slope of a corresponding fitting curve may be greater than a preset slope threshold. As another example, the plurality of feature values corresponding to the EMG signal may fluctuate abnormally when the muscle is in the over-fatigue state, and the corresponding fitting curve may have a wave shape.

It should be noted that the above descriptions of process 1100 are intended to be exemplary and illustrative only and do not limit the scope of the present disclosure. For those skilled in the art, various modifications and variations may be made to process 1100 under the guidance of the present disclosure. However, these modifications and variations remain within the scope of the present disclosure. For example, operation 1110 may be omitted, and the processing device 110 may determine the plurality of feature values based only on the plurality of second time windows. As another example, the plurality of feature values may be determined from a plurality of candidate values based on other conditions. As another example, the plurality of feature values may be evaluated based on other preset conditions to generate the running feedback information. As yet another example, operation 1130 may be omitted, and the processing device 110 may determine the plurality of feature values based on the plurality of first time windows. For illustrative purposes only, the processing device 110 may determine a candidate value for each first segmented EMG signal based on the plurality of first time windows, based on the plurality of candidate values corresponding to the first segmented EMG signals, the second segmented EMG signals may be determined and extreme values corresponding to the second segmented EMG signals may be determined by determining extreme value points, and the extreme values may be designated as the plurality of feature values.

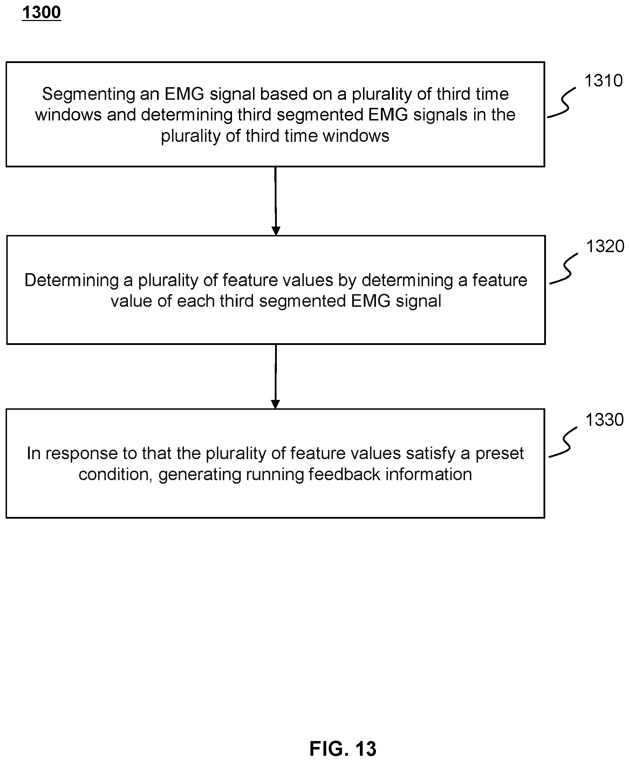

FIG. 13 is a flowchart illustrating another exemplary process for identifying a plurality of feature values of an EMG signal and generating running feedback information according to some embodiments of the present disclosure. In some embodiments, process 1300 may be performed by processing logic that may include hardware (e.g., circuitry, specialized logic, programmable logic, microcode, etc.), software (running on a processing device to execute instructions simulated by the hardware), etc., or any combination thereof. In some embodiments, one or more of the operations in the process 1300 shown in FIG. 13 may be performed by the processing device 110 and/or the one or more terminal devices 140 shown in FIG.1. For example, process 1300 may be stored in the form of instructions in the storage device 130 and called and/or executed by the processing device 110 and/or the one or more terminal devices 140. In some embodiments, operations 1020 and 1030 of process 1000 for monitoring the user's running may be performed by process 1300. In some embodiments, the process 1300 may be performed by the identification module 920 and the feedback module 930. The following takes the process 1300 performed by the processing device 110 as an example. As shown in FIG. 13, process 1300 may include the following operations.

In 1310, the EMG signal may be segmented based on a plurality of third time windows, and third segmented EMG signals in the plurality of third time windows may be determined.

In 1320, a plurality of feature values may be determined by determining a feature value of each third segmented EMG signal.

In some embodiments, the processing device 110 may segment the EMG signal based on a plurality of third time windows, determine third segmented EMG signals in the plurality of third time windows, and determine a feature value of each third segmented EMG signal. In some embodiments, the leg muscle gradually enters a fatigue state and muscle efficiency gradually decreases as a running time increases. For example, the muscle strength of the leg muscle in the fatigue state is greater than a muscle strength in a non-fatigue state when performing the same running action. As the level of fatigue increases, the muscle strength further increases. When the muscle strength is too high, the leg muscle may be injured due to over-fatigue. Therefore, the fatigue state of the leg muscle may be determined based on a change in the strength of the EMG signal.

In some embodiments, the fatigue state of the leg muscle may be determined based on an overall change in the strength of the EMG signal. Correspondingly, each of the plurality of third time windows may be a time window of any duration. By segmenting the EMG signal based on the plurality of third time windows and determining an average amplitude value (e.g., average absolute amplitude value) of each third segmented EMG signal as a feature value of the third segmented EMG signal, a plurality of feature values in a plurality of target time intervals may be determined. The average amplitude value may reflect the strength of each segmented EMG signal in each of the plurality of third time windows, such that the change in the strength of the EMG signal during running may be determined based on the plurality of feature values.

In 1330, in response to that the plurality of feature values satisfy a preset condition, running feedback information may be generated.

In some embodiments, the processing device 110 may determine whether the leg muscle is in an abnormal motion state based on the change in the strength of the EMG signal, and generate corresponding running feedback information when the abnormal motion state occurs. For example, when the muscle strength is too high, the leg muscle may be injured due to over-fatigue. Therefore, in some embodiments, the preset condition may include that the plurality of feature values include a feature value greater than a preset feature value threshold. For example, a maximal value may be determined among the plurality of feature values. The preset condition may include that the plurality of feature values include a feature value greater than the maximal value. Merely by way of example, in an initial phase (e.g., 0-40 min, 0-30 min, 0-20 min, etc.) of running, the strength of the leg muscle gradually increases to adapt to the running action and decreases after adaptation, with a maximal value of muscle strength appears during this process. Correspondingly, the plurality of feature values of the EMG signal may have a maximal value. As the running time continues to increase, the leg muscle gradually enters the fatigue state and the muscle strength gradually increases. When the muscle strength is too large (e.g., greater than the maximal value in the initial phase of running), which indicates that the muscles are overly fatigued, continuing the running action may result in an injury. In such cases, the maximal value of the plurality of feature values may be determined, and when a feature value that is greater than the maximal value appears, it may be determined that the leg muscle is over-fatigued, and fatigue feedback information may be generated. As another example, the preset condition may include that the plurality of feature values include a feature value greater than a reference threshold. The reference threshold may be determined based on historical EMG signals of historical users (e.g., a current user, users other than the current user, etc.). Merely by way of example, a plurality of historical EMG signals of a plurality of historical users may be collected, a plurality of historical feature values of each historical EMG signal in a plurality of target time intervals may be identified, and a maximum historical feature value, or 90%, 80%, 70% of the maxium historical feature value may be designated as the reference threshold. When the plurality of feature values corresponding to the EMG signal of the leg muscle of the user includes a feature value greater than the reference threshold, which indicates that the muscle strength of the user's leg muscle is relatively large and the leg muscle may be in an over-fatigue state, the fatigue feedback information may be generated.

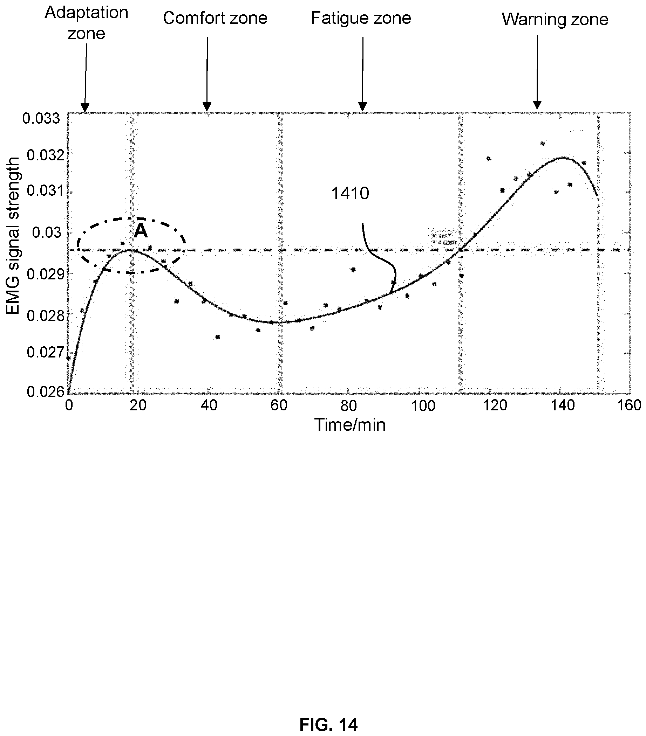

Merely by way of example, FIG. 14 is a schematic diagram illustrating a curve showing a change in a plurality of feature values of an EMG signal of a leg muscle of a user over time. The longitudinal coordinate (i.e., the feature value) of each coordinate point shown in FIG. 14 may represent an average amplitude value of candidate values in each third segmented EMG signal in a third time window, which may reflect the magnitude of the strength of the leg muscle in each third time window. The larger the longitudinal coordinate of each coordinate point, the greater the strength of the muscle. A Curve 1410 is a fitting curve generated based on the plurality of feature values. As shown in FIG. 14, a running process may be divided into a plurality of zones based on the change in the strength of the EMG signal indicated by the vertical coordinate. For example, in an adaptation zone, the muscle strength gradually increases to adapt to the running action, and the longitudinal coordinate of the curve 1410 enters a comfort zone after reaching a maximal value A, where the muscle strength begins to gradually decrease. As the running time increases, the leg muscle gradually becomes fatigued, and curve 1410 enters a fatigue zone, where the muscle strength gradually increases. When the muscle strength is too high (e.g., when the feature value is greater than the maximal value A), it may be determined that the leg muscle is over-fatigued, and continuing the running action may result in an injury to the muscle or joint. Therefore, a region where the feature value is greater than the I maximal value A may be determined as a warning zone, and the fatigue feedback information may be generated.

In some embodiments, when the muscle is over-fatigue, continuing the running action for a certain duration (e.g., 1 hour, 40 minutes, 20 minutes, 5 minutes, 30 seconds, etc.) or a certain distance (e.g., 5 km, 3 km, 500 m, etc.) may cause an injury. Therefore, a running workout amount recommendation may be generated after determining that the user is in an over-fatigue state. For example, as shown in Fig. 14, curve 1410 enters the warning zone with over-fatigue at about 110 minutes and shows an injury (e.g., cramping) at about 150 minutes, at which point the running action stops and the strength of the EMG signal gradually decreases. Therefore, it may be determined that continuing the running action for 40 minutes after the over-fatigue may cause injury. Correspondingly, the running workout amount recommendation after over-fatigue of the muscle may be to continue running for no more than 40 minutes (e.g., continue running for 20 minutes, 10 minutes, or stop immediately, etc.). In some embodiments, the running workout amount recommendation may be generated based on historical EMG signals. For example, the running workout amount recommendation for a current user may be generated based on a historical workout amount (e.g., an average, a minimum, etc., of a plurality of historical workout amounts of a plurality of historical users) corresponding to the over-fatigue of the muscle determined based on the historical EMG signals. As another example, the historical workout amount may also be combined with the current EMG signal to generate the running workout amount recommendation for the current user.

It should be noted that the above descriptions of process 1300 are intended to be exemplary and illustrative only, and do not limit the scope of application of the present disclosure. For those skilled in the art, various modifications and variations may be made to process 1300 under the guidance of the present disclosure. However, these modifications and variations remain within the scope of the present disclosure. For example, the plurality of feature values may be evaluated based on other preset conditions to generate the running feedback information.

It is to be noted that processes 1100 and 1300 of identifying a plurality of feature values of an EMG signal and generating running feedback information as described in FIGs. 11 and 13 are only exemplary illustrations, and in some embodiments, other running feedback information may be generated based on other preset conditions to generate other running feedback information. Merely by way of example, the preset condition may include conditions related to the user's running posture (e.g., running skill, balance, stability, etc.). The running feedback information may also include a target muscle strength training recommendation, running posture feedback information, or the like.

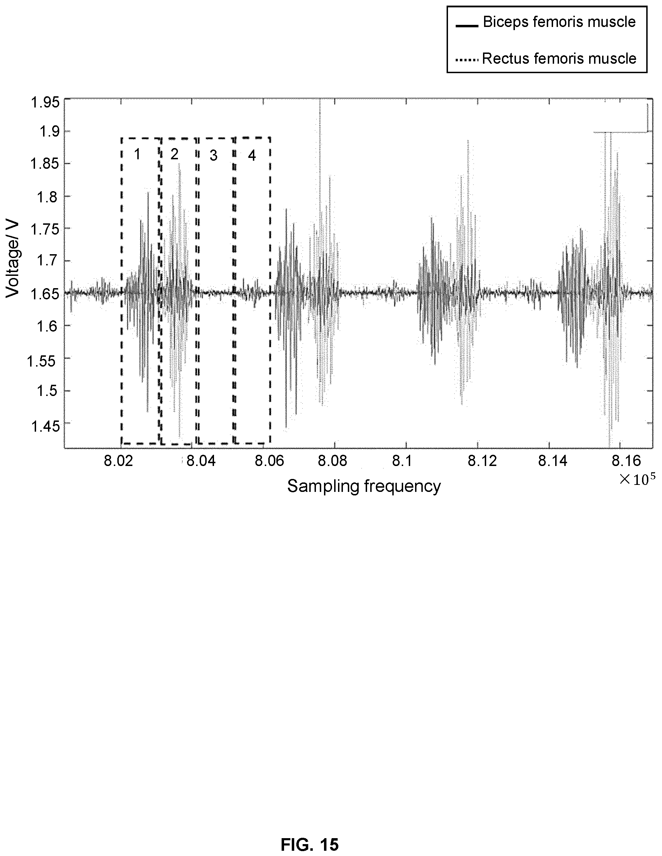

Taking the running skill as an example, FIG. 15 is a schematic diagram illustrating curves showing changes in an EMG signal of the biceps femoris muscle and an EMG signal of the rectus femoris muscle of a user over time when the user is running. As shown in FIG.15, the EMG signals in a single running cycle may be divided into four regions numbered 1-4, which correspond to four phases of the running cycle, i.e., foot contacting the ground, foot push-off, leg folding, and leg swing forward. In some embodiments, an exemplary running skill may include the biceps femoris actively exerting force during the leg folding phase of a running cycle, and correspondingly a relatively obvious EMG signal may be generated during the leg folding phase. Therefore, amplitude values (e.g., absolute amplitude values) of the EMG signal of the biceps femoris muscle during the leg folding phase may be determined as the feature values, and a preset condition associated with the running skill may include that the amplitude of the EMG signal during the leg folding phase is less than a preset amplitude threshold. When the amplitude values of the EMG signal in the leg folding phase are less than the preset amplitude threshold, it may be determined that the biceps femoris muscle is not exerting enough force in the leg folding phase, and corresponding running feedback information may be generated. For example, as shown in FIG. 15, there is no obvious fluctuation of the EMG signal of the biceps femoris at the leg folding phase in the 3rd region, and the amplitude values of the EMG signal are less than the preset amplitude threshold, therefore, it may be determined that the biceps femoris muscle is not exerting enough force in the leg folding phase. In some embodiments, an exemplary running skill may include that the biceps femoris muscle does not exert force during the leg swing forward phase of a running cycle, and correspondingly, the biceps femoris muscle does not generate an obvious EMG signal during the leg swing forward phase. Therefore, amplitude values (e.g., absolute amplitude values) of the EMG signal of the biceps femoris muscle during the leg swing forward phase may be directly determined as the feature values, and a preset condition associated with the running skill may include that the amplitude values of the EMG signal during the leg swing forward phase are greater than a preset amplitude threshold. Merely by way of example, a plurality of time intervals corresponding to the leg swing forward phase of the biceps femoris muscle may be determined as a plurality of target time intervals, and amplitude values (e.g., absolute amplitude values) of the EMG signal in the plurality of target time intervals may be determined as a plurality of feature values. When a feature value of the EMG signal of the biceps femoris muscle in a current phase is greater than the preset amplitude threshold, the biceps femoris muscle may be determined to be exerting excessive force in the current phase, and corresponding feedback information may be generated. For example, as shown in FIG. 15, the EMG signal of the biceps femoris muscle shows obvious fluctuation in the leg folding phase in the 4th region, and the amplitude values of the EMG signal in the leg folding phase are greater than the preset amplitude threshold, then it may be determined that the biceps femoris muscle is exerting excessive force in the leg folding phase.

Further, FIG. 11 and FIG. 13 and the descriptions thereof provide methods for generating running feedback information based on the EMG signal of a single leg muscle. In some embodiments, the running feedback information may be generated based on a plurality of EMG signals corresponding to a plurality of leg muscles. For example, the plurality of feature values may include an amplitude ratio of EMG signals of at least two leg muscles of the plurality of leg muscles when performing the same running action, and the preset condition may include a difference between at least two feature values (i.e., amplitude ratios) in the plurality of feature values being greater than a difference threshold. According to FIG. 15, the plurality of feature values may include an amplitude ratio (e.g., one of amplitude values, absolute amplitude values, maximum amplitude values, minimum amplitude values, average amplitude values, etc., of the two EMG signals at the same moment) of the EMG signal of the biceps femoris muscle and the EMG signal of the rectus femoris muscle when performing a same running action, and the corresponding preset condition may include that the amplitude ratio of the two EMG signals fluctuates (i.e., a difference between at least two amplitude ratios exceeding a difference threshold). When the amplitude ratio of the two EMG signals fluctuates, which indicates that the stability of at least one of the biceps femoris muscle and the rectus femoris muscle changes, running feedback information related to the stability may be generated. As another example, the plurality of feature values may include a plurality of force exertion times of a plurality of leg muscles (e.g., times when the plurality of leg muscles generate EMG signals that are greater than a preset amplitude). The corresponding preset condition may include that whether a force exertion order corresponding to the plurality of force exertion times satisfies a preset force exertion order. When the force exertion order does not satisfy the preset force exertion order, running feedback information related to the force exertion order may be generated. As yet another example, the plurality of feature values may include an amplitude value of a first EMG signal of a first muscle and an amplitude value of a second EMG signal of a second muscle (e.g., one of amplitude values, absolute amplitude values, maximum amplitude values, minimum amplitude values, average amplitude values, etc., at a same moment), and the corresponding preset condition may include that a ratio of amplitude increase speeds of the two EMG signals is greater than a ratio threshold. When the ratio of the amplitude increase speed of the first EMG signal to the amplitude increase speed of the second EMG signal is greater than a ratio threshold, which indicates that the strength of the first muscle is abnormally increased with respect to the strength of the second muscle, which further indicates that the first muscle is relatively weak. Therefore, the first muscle may be determined as the target muscle, and the target muscle strength training recommendation may be generated.

It should be noted that the above descriptions of the process are intended to be exemplary and illustrative only and do not limit the scope of the present specification. For those skilled in the art, various modifications and variations may be made to the process under the guidance of the present disclosure. However, these modifications and variations remain within the scope of the present disclosure. For example, the process described above may also include a pre-processing operation.

Having thus described the basic concepts, it may be rather apparent to those skilled in the art after reading this detailed disclosure that the foregoing detailed disclosure is intended to be presented as illustrative example and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of the present disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

As another example, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This way of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties used to describe and claim certain embodiments of the present disclosure are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameter set forth in the written description and attached claims are approximations that may vary depending upon the desired properties sought to be obtained by a particular embodiment. In some embodiments, the numerical parameter should be construed in light of the number of reported significant digits and by applying ordinary rounding techniques. Notwithstanding that the numerical ranges and parameter setting forth the broad scope of some embodiments of the present disclosure are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrating of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A signal collection device, comprising:
a wearable body; and
a plurality of electrodes disposed on the wearable body and in contact with the skin of a user, wherein
the plurality of electrodes include a first electrode and a second electrode,
the first electrode and the second electrode are configured to collect an electromyographic (EMG) signal of a muscle of the user and being spaced apart along a muscle fiber direction of the muscle,
the first electrode and the second electrode extend along an extension direction perpendicular to the muscle fiber direction, respectively, and
each of the first electrode and the second electrode has a size in the muscle fiber direction that is smaller than a size in the extension direction.

2. The signal collection device of claim 1, wherein
the plurality of electrodes further include an artifact electrode spaced apart from the first electrode along the extension direction, and
the signal collection device further includes a processing circuit configured to perform noise cancellation on a signal collected by the first electrode and a signal collected by the second electrode based on a signal collected by the artifact electrode.

3. The signal collection device of claim 2, wherein the performing the noise cancellation on the signal collected by the first electrode and the signal collected by the second electrode based on the signal collected by the artifact electrode includes:
obtaining a first signal by performing a differential operation on the signal collected by the first electrode and the signal collected by the second electrode;
obtaining a second signal by performing a differential operation on the signal collected by the first electrode and the signal collected by the artifact electrode; and
performing the noise cancellation on the first signal based on the second signal.

4. The signal collection device of claim 2, wherein
the plurality of electrodes further include a second artifact electrode spaced apart from the second electrode along the extension direction, and
the processing circuit is further configured to perform the noise cancellation on the signal collected by the first electrode and the signal collected by the second electrode based on the signal collected by the artifact electrode and a signal collected by the second artifact electrode.

5. The signal collection device of claim 4, wherein the performing the noise cancellation on the signal collected by the first electrode and the signal collected by the second electrode based on the signal collected by the artifact electrode and the signal collected by the second artifact electrode includes:
obtaining a first signal by performing a differential operation on the signal collected by the first electrode and the signal collected by the second electrode;
obtaining a second signal by performing a differential operation on the signal collected by the first electrode and the signal collected by the artifact electrode;
obtaining a third signal by performing a differential operation on the signal collected by the second electrode and the signal collected by the second artifact electrode; and
performing the noise cancellation on the first signal based on the second signal and the third signal.

6. The signal collection device of claim 2, wherein a value of a configuration parameter of the artifact electrode is different from a value of the configuration parameter of the first electrode or a value of the configuration parameter of the second electrode.

7. The signal collection device of claim 6, wherein the configuration parameter includes at least one of an area, a material, or a pressure on the skin of the user per unit of area.

8. The signal collection device of claim 1, wherein the plurality of electrodes further include:
a reference electrode, wherein the reference electrode is an integral electrode, and in the extension direction, a length of the reference electrode is greater than a length of the first electrode or a length of the second electrode.

9. The signal collection device of claim 1, wherein the plurality of electrodes further include:
a reference electrode including a plurality of sub-electrodes spaced apart and connected by wires, wherein, along the muscle fiber direction of the muscle, projections of the plurality of sub-electrodes are at least partially coincident with the first electrode or the second electrode.

10. The signal collection device of claim 1, wherein the wearable body includes a leg strap or a leg sleeve, and the plurality of electrodes are configured to collect an EMG signal of a front side or a back side of a leg of the user.

11. The signal collection device of claim 10, further including:
a plurality of additional electrodes configured to collect an EMG signal of an outer side of a thigh of the user, wherein the plurality of additional electrodes are disposed above the plurality of electrodes along a height direction of the user.

12. The signal collection device of claim 11, wherein the EMG signal of the outer side of the thigh includes an EMG signal of a tensor fascia lata.

13. The signal collection device of claim 11, wherein a ratio of a height difference between the plurality of additional electrodes and the plurality of electrodes to a length of the thigh is greater than 1/10.

14. The signal collection device of claim 1, further including:
a circuit board disposed on the wearable body, the circuit board being provided with a processing circuit configured to process signals collected by the plurality of electrodes, wherein
each of the plurality of electrodes is connected to the circuit board via a wire,
the wearable body includes a two-layer structure,
the wire is sandwiched in the two-layer structure, and
a length of the wire is configured to adapt to an elastic stretching length of the wearable body.

15. The signal collection device of claim 14, wherein for each electrode of the plurality of electrodes, the wire includes a first connection point connected to the electrode and a second connection point connected to the circuit board, and the length of the wire is greater than 110% of a straight-line distance between the first connection point and the second connection point.

16. The signal collection device of claim 14, wherein the signal collection device is configured to collect an EMG signal of a leg muscle of the user, and the processing circuit is configured to:
obtain the EMG signal of the leg muscle of the user when the user is running;
identify a plurality of feature values of the EMG signal in a plurality of target time intervals; and
in response to that the plurality of feature values satisfy a preset condition, generate running feedback information.

17. The signal collection device of claim 16, wherein the plurality of feature values include at least one of: amplitude values, absolute amplitude values, an average amplitude value, a maximum amplitude value, a minimum amplitude value, a medium value frequency, a peak frequency, a total force value, an EMG power, a foot contact time, or an average frequency, corresponding to the EMG signal in each of the plurality of target time intervals.

18. The signal collection device of claim 17, wherein the identifying a plurality of feature values of the EMG signal in a plurality of target time intervals includes:
segmenting the EMG signal based on a plurality of first time windows and determining first segmented EMG signals in the plurality of first time windows; and
determining the plurality of feature values based on the first segmented EMG signals.

19. The signal collection device of claim 18, wherein the determining the plurality of feature values based on the first segmented EMG signals includes:
determining a candidate value of each first segmented EMG signal; and
determining, from a plurality of candidate values corresponding to the first segmented EMG signals, candidate values that satisfy a condition as the plurality of feature values.

20. The signal collection device of claim 19, wherein a duration of each first time window of the plurality of first time windows is smaller than or equal to a running cycle of the user, and the candidate value of each first segmented EMG signal includes an average amplitude value of the each first segmented EMG signal.

21. The signal collection device of claim 19, wherein the determining, from a plurality of candidate values corresponding to the first segmented EMG signals, candidate values that satisfy a condition as the plurality of feature values includes:
segmenting the EMG signal based on a plurality of second time windows and determining second segmented EMG signals in the plurality of second time windows; and
determining the plurality of feature values based on candidate values of the second segmented EMG signal in each second time window.

22. The signal collection device of claim 21, wherein a duration of each second time window is larger than a duration of each first time window.

23. The signal collection device of claim 21, wherein the determining the plurality of feature values based on candidate values of the second segmented EMG signal in each second time window comprises:
for each second time window, determining at least one of a maximum value, a minimum value, an intermediate value, an average value, and a weighted average value of the candidate values of the second segmented EMG signal in the second time window as the plurality of feature values.

24. The signal collection device of claim 18, wherein the determining the plurality of feature values based on the first segmented EMG signal includes:
determining an average amplitude value of each first segmented EMG signal as the plurality of feature values.

25. The signal collection device of claim 16, wherein the preset condition includes: a feature parameter that indicates a change in the plurality of feature values being greater than a preset threshold.

26. The signal collection device of claim 16, wherein
the plurality of feature values include a maximal value, and
the preset condition includes: the plurality of feature values including a feature value greater than the maximal value.

27. The signal collection device of claim 16, wherein the running feedback information includes at least one of: a fatigue reminder, a running workout amount recommendation, or a target muscle strength training recommendation.

28. The signal collection device of claim 16, wherein
the EMG signal includes a plurality of EMG signals corresponding to a plurality of leg muscles of the user,
the plurality of feature values includes: an amplitude ratio of EMG signals of at least two leg muscles of the plurality of leg muscles when performing a same running action, and
the preset condition includes: a difference between at least two feature values in the plurality of feature values is greater than a difference threshold.

29. A method for monitoring user's running comprising:
obtaining an electromyographic (EMG) signal of a leg muscle of a user when the user is running;
identifying a plurality of feature values of the EMG signal in a plurality of target time intervals; and
in response to that the plurality of feature values satisfy a preset condition, generating running feedback information.

30. The method of claim 29, wherein the plurality of feature values include at least one of: amplitude values, absolute amplitude values, an average amplitude value, a maximum amplitude value, a minimum amplitude value, a medium value frequency, a peak frequency, a total force value, an EMG power, a foot contact time, or an average frequency corresponding to the EMG signal in each of the plurality of target time intervals.

31. The method of claim 30, wherein the identifying a plurality of feature values of the EMG signal in a plurality of target time intervals includes:
segmenting the EMG signal based on a plurality of first time windows and determining first segmented EMG signals in the plurality of first time windows; and
determining the plurality of feature values based on the first segmented EMG signals.

32. The method of claim 31, wherein the determining the plurality of feature values based on the first segmented EMG signals includes:
determining a candidate value of each first segmented EMG signal; and
determining, from a plurality of candidate values corresponding to the first segmented EMG signals, candidate values that satisfy a condition as the plurality of feature values.

33. The method of claim 32, wherein a duration of each first time window of the plurality of first time windows is smaller than or equal to a running cycle of the user, and the candidate value of each first segmented EMG signal includes an average amplitude value of the each first segmented EMG signal.

34. The method of claim 32, wherein the determining, from a plurality of candidate values corresponding to the first segmented EMG signals, candidate values that satisfy a condition as the plurality of feature values includes:
segmenting the EMG signal based on a plurality of second time windows and determining second segmented EMG signals in the plurality of second time windows; and
determining the plurality of feature values based on candidate values of the second segmented EMG signal in each second time window.

35. The method of claim 34, wherein a duration of the each second time window is larger than a duration of the each first time window.

36. The method of claim 34, wherein the determining the plurality of feature values based on candidate values of the second segmented EMG signal in each second time window includes:
for each second time window, determining at least one of a maximum value, a minimum value, an intermediate value, an average value, and a weighted average value of the candidate values of the second segmented EMG signal in the second time window as the plurality of feature values.

37. The method of claim 31, wherein the determining the plurality of feature values based on the first segmented EMG signal includes:
determining an average amplitude value of each first segmented EMG signal as the plurality of feature values.

38. The method of claim 29, wherein the preset condition includes: a feature parameter that indicates a change in the plurality of feature values being greater than a preset threshold.

39. The method of claim 29, wherein
the plurality of feature values include a maximal value, and
the preset condition includes: the plurality of feature values including a feature value greater than the maximal value.

40. The method of claim 29, wherein the running feedback information includes at least one of: a fatigue reminder, a running workout amount recommendation, or a target muscle strength training recommendation.

41. The method of claim 29, wherein
the EMG signal includes a plurality of EMG signals corresponding to a plurality of leg muscles of the user,
the plurality of feature values includes: an amplitude ratio of EMG signals of at least two leg muscles of the plurality of leg muscles when performing a same running action, and
the preset condition includes: a difference between at least two feature values in the plurality of feature values is greater than a difference threshold.
